# EUROPÄISCHE PATENTSCHRIFT

(11) **EP 2 218 795 B1**
(45) Veröffentlichungstag und Bekanntmachung des Hinweises auf die Patenterteilung: **29.06.2011**
(21) Anmeldenummer: 10154854.3
(22) Anmeldetag: 18.08.2005
(51) Int. Cl.: C12Q 1/68

(54) **Verfahren zur Unterscheidung zwischen nichtinfektiösen und infektiösen Ursachen eines Multiorganversagens**
Method for differentiating between the non-infectious and infectious causes of multiple organ failure
Procédé pour faire la différence entre des origines non infectieuses et infectieuses d'une défaillance multi-organes

(30) Priorität: 13.10.2004 DE 102004049897
(43) Veröffentlichungstag der Anmeldung: 18.08.2010
(62) Teilanmeldung aus: 05775921.9
(73) Patentinhaber: SIRS-Lab GmbH, 07745 Jena (DE)
(72) Erfinder: Russwurm, Stefan, 07743 Jena (DE); Reinhart, Konrad, 07743 Jena (DE)
(74) Vertreter: Kaiser, Jürgen

(56) Entgegenhaltungen:
- US-A1- 2004 096 917
- "AMERICA COLLEGE OF CHEST PHYSICIANS/SOCIETY OF CRITICAL CARE MEDICINE CONSENSUS CONFERENCE: DEFINITIONS FOR SEPSIS AND ORGAN FAILURE AND GUIDELINES FOR THE USE OF INNOVATIVE THERAPIES IN SEPSIS" CRITICAL CARE MEDICINE, WILLIAMS AND WILKINGS COMPANY, BALTIMORE, MA, US, Bd. 20, Nr. 6, Juni 1992 (1992-06), Seiten 864-874, XP008036965 ISSN: 0090-3493
- COBB J P ET AL: "SEPSIS GENE EXPRESSION PROFILING: MURINE SPLENIC COMPARED WITH HEPATIC RESPONSES DETERMINED BY USING COMPLEMENTARY DNA MICROARRAYS" CRITICAL CARE MEDICINE, WILLIAMS AND WILKINGS COMPANY, BALTIMORE, MA, US, Bd. 30, Nr. 12, Dezember 2002 (2002-12), Seiten 2711-2721, XP008037048 ISSN: 0090-3493
- TSUKAHARA YASUHIRO ET AL: "Gene expression in human neutrophils during activation and priming by bacterial lipopolysaccharide." JOURNAL OF CELLULAR BIOCHEMISTRY. 1 JUL 2003, Bd. 89, Nr. 4, 1. Juli 2003 (2003-07-01), Seiten 848-861, XP009063472 ISSN: 0730-2312
- PRUCHA M ET AL: "EXPRESSION PROFILING: TOWARD AN APPLICATION IN SEPSIS DIAGNOSTICS" SHOCK, Bd. 22, Nr. 1, Juli 2004 (2004-07), Seiten 29-33, XP008036997
- LIU Z ET AL: "Gene expression profiles in human nasal polyp tissues studied by means of DNA microarray" JOURNAL OF ALLERGY AND CLINICAL IMMUNOLOGY, MOSBY - YEARLY BOOK, INC, US, Bd. 114, Nr. 4, 7. Oktober 2004 (2004-10-07), Seiten 783-790, XP004631421 ISSN: 0091-6749
- DATABASE NETAFFX [Online] Affymetrix; 7. Oktober 2004 (2004-10-07), XP002372475 gefunden im HTTPS://WWW.AFFYMETRIX.COM/ANALYSIS/NETAFF X/PROBEMATCH/PROBE_MATCH.AFFX?NETAFFX=NETA FFX4_ANNOT
- RIESEWIJK A ET AL: "GENE EXPRESSION PROFILING OF HUMAN ENDOMETRIAL RECEPTIVITY ON DAYS LH+2 VERSUS LH+7 BY MICROARRAY TECHNOLOGY" MOLECULAR HUMAN REPRODUCTION, OXFORD, GB, Bd. 9, Nr. 5, Mai 2003 (2003-05), Seiten 253-264, XP008041260 ISSN: 1360-9947
- GALDERISI U ET AL: "Antisense oligonucleotides as therapeutic agents." JOURNAL OF CELLULAR PHYSIOLOGY. NOV 1999, Bd. 181, Nr. 2, November 1999 (1999-11), Seiten 251-257, XP009063561 ISSN: 0021-9541
- JANSEN B ET AL: "Antisense therapy for cancer-the time of truth" LANCET ONCOLOGY, LANCET PUBLISHING GROUP, LONDON, GB, Bd. 3, Nr. 11, November 2002 (2002-11), Seiten 672-683, XP004810743 ISSN: 1470-2045
- RAY A ET AL: "PEPTIDE NUCLEIC ACID (PNA): ITS MEDICAL AND BIOTECHNICAL APPLICATIONS AND PROMISE FOR THE FUTURE" FASEB JOURNAL, FED. OF AMERICAN SOC. FOR EXPERIMENTAL BIOLOGY, BETHESDA, MD, US, Bd. 14, Nr. 9, 1. Juni 2000 (2000-06-01), Seiten 1041-1060, XP001162782 ISSN: 0892-6638

## Beschreibung

Die vorliegende Erfindung betrifft ein Verfahren zur in vitro Messung von Genexpressionsprofilen unter Verwendung spezifischer Gene sowie Genfragmenten davon, wobei die Genaktivität einer Mehrzahl von Genen aus Proben von Patienten bestimmt wird, um das Vorliegen einer nichtinfektiösen oder infektiösen Ursache eines Multiorganversagens festzustellen, gemäß Anspruch 1.

Weiterhin betrifft die vorliegende Erfindung neue Möglichkeiten der Unterscheidung zwischen nichtinfektiösen und infektiösen Ursachen eines Multiorganversagens von Patienten, die sich aus experimentell abgesicherten Erkenntnissen im Zusammenhang mit dem Auftreten von Änderungen der Genaktivitäten (Transkription) bei Patienten mit Multiorganversagen ableiten lassen.

Trotz Fortschritten im pathophysiologischen Verständnis und der supportiven Behandlung stellt das Multiorgandysfunktionssyndrom (MODS) bzw. das Multiorganversagen (MOV) bei intensivmedizinischen Patienten die häufigste Todesursache dar und nimmt weltweit weiter zu. Die Folgen dieser Entwicklung sind nicht nur für den einzelnen Patienten erheblich, sondern haben enorme Auswirkungen auf die Kosten des Gesundheitswesens und den medizinischen Fortschritt in vielen Bereichen der Medizin.

Als Multiorganversagen bezeichnet man das gleichzeitig oder in rascher zeitlicher Abfolge auftretende Versagen von zwei oder mehr vitalen Organsystemen. Das Multiorgandysfunktionssyndrom (MODS) geht als initiale Organinsuffizienz dem MOV voraus [1]. Man spricht heute vom Multiorganversagen wenn zwei oder mehr Organe gleichzeitig oder nacheinander Funktionstörungen aufweisen, wobei ein chronisch persistierendes Organversagen auszuschließen ist [2]. Die Prognose des MOV hängt eng mit der Anzahl der beteiligten Organsysteme zusammen. Die Mortalität beträgt bei Versagen eines Organs innerhalb der ersten 24 Stunden 22%, nach 7 Tagen 41 %. Beim Versagen von drei Organsystemen steigt die Mortalität am ersten Tag auf 80 % und nach 4 Tagen auf 100 % an [3].

Zur klinischen Schweregradeinteilung des MODS und MOV wird regelmäßig der Multiple-Organ-Failure-Score (MOF-Score) von GORIS et al. [4] oder alternativ auch der Sepsis-related Organ Failure Assessment-(SOFA-) Score verwendet [5]. Der MOF-Score erlaubt eine schnelle und klinisch einfache Klassifikation der Organfunktion in drei Abstufungen. Ein MOF-Score > 4 wird in der klinischen Literatur regelmäßig als MOV bezeichnet [6]. Der SOFA-Score ist ein Punktesystem, welches die schnelle klinische Beurteilung der Funktion, jeweils in vier Schweregradstufen, folgende Organsysteme bewertet: Atmung (Lunge), Koagulation, Leber, Herz-Kreislaufsystem, zentrales Nervensystem und Niere.

Das MOV läuft klinisch in drei Phasen ab [7]:
1. Organ im Schock: Der auslösende pathophysiologische Mechanismus ist ein Perfusionsdefizit unterschiedlichster Genese. Dieses Geschehen spielt sich innerhalb von Stunden ab und hinterläßt noch keine bleibenden Schäden.
2. Organdysfunktion: Ein fortbestehendes Perfusionsdefizit innerhalb der nächsten Tage führt zur Entstehung eines SIRS (Systemic Inflammatory Response Syndrome, klassifiziert nach [8]) mit lokalen Ödemen und Zellschädigungen. Diese Phase wird als Multiorgandysfunktionssyndrom (MODS) bezeichnet.
3. Organversagen: Das fortbestehende Perfusionsdefizit führt zur Stase im Splanchnikusgebiet, wodurch es zur Superinfektion und Translokation von Endotoxinen aus dem Darm kommt. Das führt zu einer Potenzierung der klinischen Symptome und zum Vollbild der Sepsis. Aus der Organdysfunktion wird ein Organversagen.

MODS und MOV sind Krankheitsbilder mit einer komplizierten Pathophysiologie. Bis heute sind die genauen molekularen Ursachen der Entstehung und die Komplexizität der immunologisch-inflammatorischen Wirtsantwort auf schwere Infektion und Trauma, die zur Auslösung eines SIRS und zu den entsprechenden kardiozirkulatorischen Auswirkungen führen kann, nur unzureichend verstanden [9].

MODS und MOV können kann sowohl infektiologischer als auch nichtinfektiologischer Genese sein. MODS und MOV treten regelmässig als klinisch wichtige Komplikation bei Patienten mit Sepsis, nach traumatischen Schock, bei Patienten nach Operationen unter Einsatz der Herz-Lungen-Maschine, nach Organtransplantationen u.v.m. auf (Abbildung 1). Ein wichtiger Pathomechanismus für die Entstehung von MODS und MOV ist die Entwicklung eines systemischen Inflammationssyndromes (SIRS, [8]). Die im Rahmen eines SIRS initiierten pathophysiologischen Prozesse involvieren nicht nur alle Komponenten des Immunsystems, sondern beeinträchtigen das kardiozirkulatorische System in allen Ebenen und beschränken sich nicht nur auf Myokarddepression und Vasodilatation. Die kardiozirkulatorischen Veränderungen vor allem auf Ebene der Mikrozirkulation bilden die gemeinsame Endstrecke und resultieren in einer Gewebehypoxie, die als wichtiger Kofaktor in der Pathogenese des Multiorganversagens gilt.

Abbildung 1 beschreibt exemplarisch die aus heutiger Sicht wichtigsten Mechanismen der Entstehung von MODS und MOV [10]: Ein überaktives Immunsystem scheint bei der Entstehung des Multiorganversagens eine zentrale Rolle zu spielen. Dabei nimmt das Endothel durch Sekretion von Zytokinen und durch Vermittlung der Leukozyten-Adhäsion eine zentrale Schlüsselrolle ein. In den Endothelzellen werden Signaltransduktionskaskaden aktiviert, die letztendlich zur Expression und Aktivierung von Transkriptionsfaktoren führen.

Das noch lückenhafte Wissen über die Abläufe in der Frühphase des MODS und MOV ist der Grund dafür, das es bis heute keine sensitive/spezifische Diagnostik, die zwischen infektiösen und nichtinfektiösen Ursachen diskriminieren kann, existiert. Neuartige Biomarker und Diagnostika, nunmehr auch auf Genexpressionsebene, können die für die Früherkennung des Multiorganversagens sowie zur Unterscheidung zwischen infektiösen und nichtinfektiösen Ursachen eines MODS und MOV essentiellen diagnostischen Informationen liefern und stellen darüber hinaus einen wichtigen Beitrag zur Aufklärung der pathophysiologischen Mechanismen systemischer Inflammationen dar.

Die klinisch häufig benutzten Frühsymptome wie Fieber, Leukozytose, Tachykardie und Tachypnoe sind bei der Diagnose eines MODS oder MOV sowie bei der Unterscheidung zwischen infektiösen und nichtinfektiösen Ursachen eines MODS und MOV völlig unspezifisch. Parameter, welche die Mikrozirkulationsstörungen früh erfassen, wie pH-Veränderungen der Drammukosa [11] und Laktatspiegel im Kapillarbett [12,13], das Auftreten einer respiratorischen Insuffizienz, deren Ursache nicht in der Lunge liegt [2],der Anstieg der Leukozyten-Elastase [14,15], die Höhe des Neopterin-Spiegels [16], die Aktivierung polymorphkerniger Leukozyten und die Höhe des IL-6-Spiegels [17] sind als Frühparameter für die spätere Entstehung von MODS und MOV bedingt geeignet, können aber keinen Beitrag zur Unterscheidung zwischen infektiösen und nichtinfektiösen Ursachen eines MODS und MOV machen. Es besteht daher ein dringender Bedarf für neue diagnostische Verfahren, welche die Fähigkeit des Fachmanns verbessern sollen, nichtinfektiöses von infektiösen MODS oder MOV frühzeitig zu unterscheiden, und dem Ansprechen auf spezifische Behandlungen Aussagen abzuleiten.

Genau die Unterscheidung zwischen infektiösen und nichtinfektiösen Ursachen eines MODS und MOV ist aber von höchster medizinischer Bedeutung, da mittels einer solchen Unterscheidung z.B. Antibiotika spezifischer eingesetzt werden können, was neben einer Vermeidung von Nebenwirkungen durch den unspezifischen Einsatz von Antibiotika auch zu einer erheblichen Kosteneinsparung beiträgt. Gleichfalls können so den Patienten sehr belastende sowie Zeit- und Personal-intensive diagnostische Massnahmen (z.B. Transport zum CT/MRT) zur Identifizierung des jeweiligen Infektionsortes, die Durchführung umfangreicher mikrobiologischer Methoden (z.B. die Untersuchung von Blutkulturen, die ebenfalls mit der Abnahme großer Mengen an Blut verbunden sind) aber auch der risikoreiche Austausch aller mit dem Patienten verbundener Plastikmaterialien wie Venenkatheder etc. bei Vorliegen eines nichtinfektiösen MODS oder MOV vermieden werden. Vice versa kann die schnelle Identifikation infektiöser Ursachen, eines MODS oder MOV die zeitnahe Einleitung solcher Maßnahmen und damit die Reduktion der Sterblichkeit sicherstellen.

US2004/0096917 offenbart ein Verfahren zur Feststellung einer Sepsis durch das Isolieren kleiner Moleküle aus Patientenproben, sowie deren Analyse mittels GC/MS wodurch individuelle Biomarkerprofile erhalten werden. Im Rahmen dessen wird der Zusammenhang zwischen Sepsis, septischem Schock und multiplem Organversagen beschrieben, jedoch enthält die Veröffentlichung keinen Hinweis auf eine Genexpressionsanalyse zur Unterscheidung zwischen infektiösem und nichtinfektiösem Multiorganversagen.

Technologische Fortschritte, insbesondere die Entwicklung der Microarray-Technologie, versetzen den Fachmann nun in die Lage, 10000 oder mehr Gene und deren Genprodukte gleichzeitig zu vergleichen. Die Anwendung solcher Microarray-Technologien kann nun Hinweise auf den Status von Gesundheit, Regulationsmechanismen, biochemischer Wechselwirkungen und Signalübertragungsnetzwerken geben. Das Verbessern des Verständnisses darüber, wie ein Organismus auf Infektionen reagiert, sollte die Entwicklung von verstärkten Erkennungs-, Diagnose- und Behandlungsmodalitäten für Sepsis-Erkrankungen erleichtern.

Microarrays stammen vom "Southern blotting" [19] ab, was die erste Herangehensweise darstellt, DNA-Moleküle in einer räumlich ansprechbaren Art und Weise auf einer festen Matrix zu immobilisieren. Die ersten Microarrays bestanden aus DNA-Fragmenten, oft mit unbekannter Sequenz, und wurden auf eine poröse Membran (normalerweise Nylon) punktweise aufgebracht. Routinegemäß wurden cDNA, genomische DNA oder Plasmid-Bibliotheken verwendet, und das hybridisierte Material wurde mit einer radioaktiven Gruppe markiert [20-22].

Kürzlich hat es die Verwendung von Glas als Substrat und Fluoreszenz zur Detektion zusammen mit der Entwicklung neuer Technologien für die Synthese und für das Aufbringen der Nukleinsäuren in sehr hohen Dichten erlaubt, die Nukleinsäurearrays zu miniaturisierten bei gleichzeitiger Erhöhung des experimentellen Durchsatzes und des Informationsgehaltes [23-25].

Weiterhin ist aus WO 03/002763 bekannt, dass die Messung der Genexpression mittels Microarrays grundsätzlich für die Diagnose von Sepsis und sepsisähnlichen Zuständen verwendet werden können.

Eine Begründung für die Anwendbarkeit der Microarray-Technologie wurde zunächst durch klinische Untersuchungen auf dem Gebiet der Krebsforschung geliefert. Hier haben Expressionsprofile ihre Nützlichkeit bei der Identifizierung von Aktivitäten einzelner Gene oder Gengruppen gezeigt, die mit bestimmten klinischen Phänotypen korrelieren [26]. Durch die Analyse vieler Proben, die von Individuen mit oder ohne akute Leukämie oder diffusen B-Zell Lymphomen stammten, wurden Genexpressionsmarker (RNA) gefunden und anschließend für die klinisch relevante Klassifizierung dieser Krebsarten angewandt [26,27]. Golub et al. haben herausgefunden, daß verlässliche Vorhersagen nicht aufgrund von irgendeinem einzelnen Gen gemacht werden können, aber daß Vorhersagen, die auf der Veränderung der Transkritiption von 53 Genen (ausgewählt aus über 6000 Genen, die auf den Arrays vertreten waren) basieren, sehr genau sind [26].

Alisadeh et al. [27] untersuchten große B-Zell Lymphome (DLBCL). Die Autoren erarbeiteten Expressionsprofile mit einem "Lymphochip", einem Microarray, der 18 000 Klone komplementärer DNA trug und entwickelt worden war, um Gene zu überwachen, die in normale und abnormale Lymphozytenentwicklung involviert sind. Unter Anwendung von Cluster-Analysen waren sie in der Lage, DLBCL in zwei Kategorien einzuteilen, welche starke Unterschiede bezüglich der Überlebenschancen der Patienten aufzeigten. Die Genexpressionsprofile dieser Untergruppen entsprachen zwei bedeutsamen Stadien der B-Zelldifferenzierung.

Die grundsätzliche Verwendbarkeit von Genexpressionsprofilen, welche beispielsweise mittels der Microarray-Technik erhalten werden können, zur Diagnose von SIRS, generalisierten inflammatorischen Entzündungen, Sepsis und schwerer Sepsis ist in den nicht vorveröffentlichten deutschen Patentanmeldungen der Anmelderin der vorliegenden Erfindung DE 103 40 395.7, DE 103 36 511.7, DE 103 150 31.5 sowie 10 2004 009 952.9, auf die hiermit vollinhaltlich Bezug genommen wird, beschrieben.

Von Feezor et al. [28] ist bekannt, dass sich die Genaktivitäten von Patienten, welche aufgrund ihrer operativen Behandlung ein SIRS mit Multiorgan Dysfunction Syndrome (MODS) entwickelten, gegenüber Patienten, die unter den gleichen operativen Bedingungen eine SIRS ohne MODS entwickelten, unterscheiden. Diese Untersuchungen lassen jedoch keine Aussage über die Unterscheidung von nichtinfektiösen MOV gegenüber infektiösen MOV zu, da in bei den Patienten keine Infektion nachgewiesen wurde.

Weiterhin beschreiben Cobb et al. [29] die Verwendung der Mikroarraytechnologie zur Feststellung einer polymikrobiellen abdominalen Sepsis bei Mäusen. Tsukahara et al. [30] untersuchten unter Verwendung von Oligonukleotidchips Genexpressionsmuster in menschlichen neutrophilen Granulozyten, die mit den *E*. *coli* Lipopolysacchariden stimuliert waren. In keiner dieser Veröffentlichungen wird jedoch ein Verfahren beschrieben, dass die Unterscheidung zwischen infektiösem und nichtinfektiösen Multiorganversagen ermöglicht.

Die Verwendung von Genexpressionsprofilen zur Unterscheidung zwischen einem nichtinfektiösen MOV und einem infektiösen MOV wurde noch nicht beschrieben.

Ausgangspunkt für die in der vorliegenden Patentanmeldung offenbarten Erfindung ist die Erkenntnis, daß sich die Genaktivitäten von Patienten mit nichtinfektiösem MOV von Genaktivitäten von Patienten mit infektiösem MOV unterscheiden. Diese Unterschiede der Genaktivitäten lassen es somit zu, anhand der Genexpression zwischen nichtinfektiösen und infektiösen MOV zu unterscheiden. Diese Unterscheidung ist mit den bisher zur Diagnose verwendeten klinischen Parametern nicht möglich, aber für die Einleitung einer spezialisierten intensivmedizinischen Therapie sehr bedeutungsvoll.

Der vorliegenden Erfindung liegt somit die Aufgabe zugrunde, durch die Verwendung von Genaktivitätsmarkern zwischen einem nichtinfektiösen MOV und einem infektiösen MOV zu unterscheiden.

Diese Aufgabe wird durch die Merkmale des Anspruchs 1 gelöst.

Die vorliegende Erfindung betrifft ein Verfahren gemäß Anspruch 1.

Ferner dient die vorliegende Offenbarung der therapiebegleitenden Verlaufsbeurteilung von Patienten, welche an nichtinfektiösen und infektiösen Ursachen eines Multiorganversagens erkrankt sind.

Die vorliegende Erfindung ist ferner nützlich als Ein- oder Ausschlußkriterium von Patienten, die an nichtinfektiösen oder infektiösen Ursachen eines Multiorganversagens erkrankt sind, in klinische Studien der Phasen 2-4.

Eine bevorzugte Ausführungsform der Offenbarung liegt in der Erstellung von Genaktivitätsdaten für die elektronische Weiterverarbeitung sowie zur Herstellung von Software für die Beschreibung der individuellen Prognose eines Sepsispatienten, für Diagnosezwecke und/oder Patientendatenmangementsystemen.

Die vorliegende Erfindung kann auch zur Herstellung von "in silico" Expertensystemen und/oder zur "in silico"-Modellierung von zelluläreren Signalübertragungswegen verwendet werden.

Zur Erstellung des Genexpressionsprofiles gemäß der vorliegenden Erfindung wird eine Mehrzahl von spezifischen Genen und/oder Genfragmenten verwendet, welche ausgewählt werden aus der Gruppe bestehend aus SEQ-ID No. 1 bis SEQ-ID No. 1297 sowie Genfragmenten davon mit wenigstens 20-2000, bevorzugt 20-200, mehr bevorzugt 20-80 Nukleotiden.

Diese Sequenzen mit der Sequenz ID: 1 bis zur Sequenz ID: 1297 sind durch den Umfang der vorliegenden Erfindung mit umfaßt und sind dem angefügten, 1297 Sequenzen umfassenden, Sequenzprotokoll, das somit Bestandteil der Beschreibung der vorliegenden Erfindung ist, im Einzelnen offenbart und ist somit ebenfalls Bestandteil der Offenbarung der Erfindung. Dieses Sequenzprotokoll beinhaltet zudem eine Zuordnung der einzelnen Sequenzen mit der Sequenz ID: 1 bis zur Sequenz ID: 1297 zu deren GenBank Accession Nr. (Internet-Zugang über http://www.ncbi.nlm.nih.gov/).

Darüber hinaus kann die vorliegende Offenbarung in vitro aus einer Patientenprobe erhaltene Genexpressionsprofile und/oder die hierfür verwendeten Sonden, welche ausgewählt werden aus der Gruppe bestehend aus SEQ-ID No. 1 bis SEQ-ID No. 1297 sowie Genfragmenten davon mit wenigstens 20-2000, bevorzugt 20-200, mehr bevorzugt 20-80 Nukleotiden, zum Ausschalten und/oder zur Aktivitätsveränderung von Zielgenen und/oder zur Bestimmung der Genaktivität zum Screening von Wirkstoffen gegen nichtinfektiöses/infektiöses Multiorganversagen und/oder zur Beurteilung der Wirkung gegen nichtinfektiöses/infektiöses Multiorganversagen, verwenden.

Ferner kann man bei Patienten, die an nichtinfektiösen oder infektiösen Ursachen eines Multiorganversagens erkrankt sind, in klinische Studien der Phasen 2-4 die Genaktivitäten in einer biologischen Flüssigkeit bestimmen und aus deren "Wert" Schlüsse hinsichtlich des Krankheitsverlaufs, der Überlebenswahrscheinlichkeit, des Therapieverlaufs oder der Ein- oder Ausschlussmöglichkeit dieser Patienten für klinische Studien ziehen.

Für die Zwecke der vorliegenden Erfindung wird ein spezifisches Gen und/oder Genfragment aus der Gruppe bestehend aus SEQ-ID No. 1 bis SEQ-ID No. 1297 sowie Genfragmenten davon mit wenigstens 20-2000, bevorzugt 20-200, mehr bevorzugt 20-80 Nukleotiden, ausgewählt.

Eine weitere Ausführungsform der Erfindung ist dadurch gekennzeichnet; dass wenigstens 2 bis 100 unterschiedliche Gene und/oder Genfragmente verwendet werden.

Eine weitere Ausführungsform der Erfindung ist dadurch gekennzeichnet, daß wenigstens 200 unterschiedliche Gene und/oder Genfragmente verwendet werden.

Eine weitere Ausführungsform der Erfindung ist dadurch gekennzeichnet, daß wenigstens 200 bis 500 unterschiedliche Gene und/oder Genfragmente verwendet werden.

Eine weitere Ausführungsform der Erfindung ist dadurch gekennzeichnet, daß wenigstens 500 bis 1000 unterschiedliche Gene und/oder Genfragmente verwendet werden.

Eine weitere Ausführungsform der Erfindung ist dadurch gekennzeichnet, daß wenigstens 1000 bis 2000 unterschiedliche Gene und/oder Genfragmente verwendet werden.

Eine weitere Ausführungsform der Offenbarung ist dadurch gekennzeichnet, daß die in Anspruch 1 aufgelisteten Gene oder Genfragmente und/oder von deren RNA abgeleiteten Sequenzen ersetzt werden durch synthetische Analoga, Aptamere sowie Peptidonukleinsäuren.

Eine weitere Ausführungsform der Offenbarung ist dadurch gekennzeichnet, daß die synthetischen Analoga der Gene 5-100, insbesondere ca. 70 Basenpaare umfassen.

Eine weitere Ausführungsform der Erfindung ist dadurch gekennzeichnet, daß die Genaktivitäten mittels Hybridisierungsverfahren bestimmt wird.

Eine weitere Ausführungsform der Erfindung ist dadurch gekennzeichnet, daß die Genaktivität mittels Microarrays bestimmt wird.

Eine weitere Ausführungsform der Erfindung ist dadurch gekennzeichnet, daß die Genaktivität durch Hybridisierungs-unabhängige Verfahren, insbesondere enzymatische und/oder chemische Hydrolyse und/oder Amplifikationsverfahren, vorzugsweise PCR, anschließende Quantifizierung der Nukleinsäuren und/oder von Derivaten und/oder Fragmenten derselben, bestimmt wird.

Eine weitere Ausführungsform der Erfindung ist dadurch gekennzeichnet, daß die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

Eine weitere Ausführungsform der Erfindung ist dadurch gekennzeichnet, daß Zellproben gegebenenfalls einer lytischen Behandlung unterzogen werden, um deren Zellinhalte freizusetzen.

Es ist dem Fachmann klar, daß die in den Ansprüchen dargelegten einzelnen Merkmale der Erfindung ohne Einschränkung beliebig miteinander kombinierbar sind.

Als Markergene im Sinne der Offenbarung werden alle abgeleiteten DNA-Sequenzen, Partialsequenzen und synthetischen Analoga (beispielsweise Peptido-Nukleinsäuren, PNA) verstanden. Die auf Bestimmung der Genexpression auf RNA-Ebene bezogene Beschreibung der Erfindung stellt keine Einschränkung sondern nur eine beispielhafte Anwendung dar.

Die auf Blut bezogene Beschreibung der Erfindung stellt nur eine beispielhafte Anwendung der Erfindung dar. Als biologische Flüssigkeiten im Sinne der Erfindung werden alle Körperflüssigkeiten des Menschen verstanden.

Weitere Vorteile und Merkmale der vorliegende Erfindung ergeben sich aufgrund der Beschreibung eines Ausführungsbeispiels, sowie anhand der Zeichnung.

### Ausführungsbeispiel

Untersuchungen der differentiellen Genexpression zur Unterscheidung zwischen nichtinfektiösen und infektiösen Ursachen eines Multiorganversagens.

Für die Messung der differentiellen Genexpression zur Unterscheidung zwischen nichtinfektiösen und infektiösen Ursachen eines Multiorganversagens wurden Untersuchungen von Vollblutproben von insgesamt 57 Patienten, welche auf operativen Intensivstationen behandelt wurden, durchgeführt.

Es wurden Vollblutproben von 31 Patienten abgenommen, welchen im Rahmen ihrer intensivmedizinischen Betreuung ein infektiöses MOV [dann als schwere Sepsis oder septischer Schock bezeichnet und klassifiziert nach 8] entwickelten.

Weiterhin wurden Vollblutproben von 26 Patienten abgenommen, welche im Rahmen ihrer intensivmedizinischen Betreuung ein nichtinfektiöses MOV [klassifiziert nach 8] entwickelten.

Als Referenzproben dienten die totale RNA aus Zelllinien SIG-M5.

Ausgewählte Charakteristika der beiden Patientengruppen sind in der Tabelle 1 dargestellt. Dabei werden Angaben zum Alter, Geschlecht, und dem SOFA-Score als Maß für die Funktion der Organsysteme gemacht. Gleichfalls sind die Plasmaproteinspiegel von Procalcitonin (PCT) und CRP, die Zahl der Leukozyten sowie die häufigsten CDC-Kriterien (Center of Disease Control) der Patienten angegeben.

Alle Patientenproben wurden mit der Referenzprobe jeweils auf einem Microarray ko-hybridisiert.

**Tabelle1: Daten der Patientengruppen**

| | **Patienten mit infektiösem MOV** | **Patienten mit nichtinfektiösem** MOV |
|---|---|---|
| **Anzahl** | 31 | 26 |
| **Geschlecht m/w** | 17/14 | 15/11 |
| **Alter* [Jahre]** | 60 (17) | 69 (10) |
| **APACHE-II-Score* [Punkte]** | 14 (10) | 14,9 (3,4) |
| **SOFA-Score* [Punkte]** | 10 (3) | 8 (3) |
| **Anzahl von OD*** | 3 (1) | 3 (1) |
| **PCT* [ng/ml]** | 3,1 (7,7) | 3.8(6.7) |
| **CRP* [**µ**g/l]** | 188 (168) | 80.2 (90.2) |
| **Leukozyten* [Anz./l]** | 13.00(8 150) | 12.300 (6925) |
| **Art der Infektion It. CDC Kriterium:** | | |
| ***Pneumonie*** | 15 Patienten | |
| ***Intraabdominelle Infektion*** | 13 Patienten | keine |
| ***Infektion OP-Ort ohne Wundinfektion*** | 2 Patienten | |
| ***Infektion des Gastrointestinaltrakt*** | 1 Patient | |

| | | |
|---|---|---|
| * Median (Intraquartilabstand) | | |

### Experimentelle Beschreibung:

Nach Abnahme des Vollblutes wurde die totale RNA der Proben unter Anwendung des PAXGene Blood RNA Kit gemäß den Vorgaben des Herstellers (Qiagen) isoliert.

### Zellkultivierung

Für die Zellkultivierung (Kontrollproben) wurden 19 Kryozellkulturen (SIGM5) (eingefroren in flüssigem Stickstoff) genutzt. Die Zellen wurden jeweils mit 2 ml Iscove's Medium (Biochrom AG) beimpft ergänzt mit 20% fetalen Kälber Serum (FCS). Die Zellkulturen wurden anschliessend für 24 Stunden bei 37° C unter 5% CO2 in 12-well Platten inkubiert. Danach wurde der Inhalt von 18 Wells in 2 Teile mit jeweils dem gleichen Volumen geteilt, sodass schliesslich 3 Platten des gleichen Formats (insgesamt 36 Wells) zur Verfügung standen. Die Kultivierung wurde anschliessend für 24 Stunden unter den gleichen Bedingungen fortgeführt. Im Anschluss daran wurden die resultierenden Kulturen von 11 Wells jeder Platte vereint und zentrifugiert (1000 x g, 5 min, Raumtemperatur). Der Überstand wurde verworfen und das Zellpellet in 40 ml des o.g. Mediums gelöst. Diese 40 ml gelöste Zellen wurden in zwei 250 ml Kolben zu gleichen Teilen aufgeteilt und nach 48 Stunden Inkubation und Zugabe von 5 ml des o.g. Mediums wiederum inkubiert. Von den restlichen 2 ml der zwei verbleibendenden Platten wurden 80 µl in leere Wells der gleichen Platten gegeben, welche bereits vorher mit 1 ml des o.g. Mediums präpariert waren. Nach 48 Stunden Inkubation wurde nur eine der 12 Well-Platten wie folgt prozessiert: Aus jedem Well wurden 500 µl entnommen und vereint. Die daraus resultierenden 6 ml wurden in einen 250 ml Kolben gegeben, welcher ca. 10 ml frisches Medium enthielt. Dieses Gemisch wurde mit 1000 x g 5 Minuten bei Raumtemperatur zentrifugiert und in 10 ml des o.g. Mediums gelöst. Die anschliessende Zellzählung ergab folgendes Ergebnis: 1,5 x 107 Zellen pro ml, 10 ml Gesamtvolumen, Gesamtzahl der Zellen: 1,5 x 108. Da die Zellzahl noch nicht ausreichend war, wurden 2,5 ml des o.g. Zellsuspension in 30 ml des o.g. Mediums in einen 250 ml (75 cm²) Kolben gegeben (insgesamt 4 Kolben). Nach 72 Sunden Inkubationszeit wurden jeweils 20 ml frischen Mediums in die Kolben gegeben. Nach folgender 24-stündiger Inkubation erfolgte die Zellzählung wie oben beschrieben, die eine Gesamtzellzahl von 3,8 x 10⁸ Zellen ergab. Um die gewünschte Zellzahl von 2 x 106 Zellen zu errreichen wurden die Zellen in 47,5 ml des o.g. Mediums in 4 Kolben resuspendiert. Nach einer Inkubationszeit von 24 Stunden wurden die Zellen zentrifugiert und zweimal mit Phosphatpuffer ohne Ca²⁺ und Mg²⁺ (Biochrom AG) gewaschen.

Die Isolation der totalen RNA erfolgt mittels des NucleoSpin RNA L Kits (Machery&Nagel) entsprechend den Angaben des Herstellers. Die oben beschriebene Prozedur wurde wiederholt bis die erforderliche Zellzahl erreicht wurde. Dies war erforderlich, um die erforderliche Menge von 6 mg totale RNA zu erreichen, was etwa einer Effizienz von 600 µg RNA pro 108 Zellen entspricht.

### Reverse Transkription / Markierung / Hybridisierung

Nach Abnahme des Vollblutes wurde die totale RNA der Proben unter Verwendung des PAXGene Blood RNA Kits (PreAnalytiX) gemäss den Vorgaben des Herstellers isoliert und auf ihre Qualität geprüft. Von jeder Probe wurden 10 µg totale RNA aliquotiert und zusammen mit 10 µg total RNA aus SIGM5-Zellen als Referenz-RNA zu komplementärer DNA (cDNA) mit der reversen Transkriptase Superscript II (Invitrogen) umgeschrieben und die RNA anschließend durch alkalische Hydrolyse aus dem Ansatz entfernt. Im Reaktionsansatz wurde ein Teil des dTTP durch Aminoallyl-dUTP (AA-dUTP) ersetzt, um später die Kopplung des Fluoreszenzfarbstoffes an die cDNA zu ermöglichen.

Nach der Aufreinigung des Reaktionsansatzes wurden die cDNA der Proben und Kontrollen mit den Fluoreszenzfarbstoffen Alexa 647 und Alexa 555 kovalent markiert und auf einem Microarray der Firma SIRS-Lab hybridisiert. Auf dem verwendeten Microarray befinden sich 5308 immobilisierte Polynukleotide mit einer Länge von 55 - 70 Basenpaaren, die jeweils ein humanes Gen repräsentieren und Kontrollspots zur Qualitätssicherung. Ein Microarray unterteilt sich in 28 Subarrays mit einem Raster von 15x15 Spots.

Die Hybridisierung und das anschliessende Waschen bzw. Trocknen wurde in der Hybridisierungsstation HS 400 (Tecan) nach Angaben des Herstellers über 10,5 Stunden bei 42°C durchgeführt. Die verwendete Hybridisierungslösung besteht aus den jeweiligen gelabelten cDNA-Proben, 3,5x SSC (1x SSC enthält 150 mM Natriumchlorid und 15 mM Natriumcitrat), 0,3% Natriumdodecylsulfat (V/V) 25% Formamid (V/V) und je 0,8 µg µl-1 cot-1 DNA, Hefe t-RNA und poly-A RNA. Das anschliessende Waschen der Mikroarrays wurde mit nachfolgendem Programm bei Raumtemperatur in durchgeführt: je 90 Sekunden spülen mit Waschpuffer 1 (2x SSC, 0,03% Natriumdodecylsulfat), mit Waschpuffer 2 (1x SSC) und abschließend mit Waschpuffer 3 (0,2x SSC). Danach wurden die Mikroarrays unter einem Stickstoffstrom mit einem Druck von 2,5 bar bei 30 °C über 150 Sekunden getrocknet.

Nach der Hybridisierung wurden die Hybridisierungssignale der Mikroarrays mit einem GenePix 4000B Scanner (Axon) ausgelesen und die Expressionsverhältnisse der differenziert exprimierten Gene mit der Software GenePix Pro 4.0 (Axon) bestimmt.

### Auswertung:

Für die Auswertung wurde die mittlere Intensität eines Spots als der Medianwert der zugehörigen Spotpixel bestimmt.

### Korrektur systematischer Fehler:

Die Korrektur systematischer Fehler erfolgte nach dem Ansatz von Huber et al. [31]. Dabei wurden der additive und der multiplikative Bias innerhalb eines Microarrays aus 70% der vorhandenen Genproben geschätzt. Für alle weiteren Berechnungen wurden die Signale mittels arcus sinus hyperbolicus transformiert.

Für die Analyse wurden die normalisierten und transformierten relativen Verhältnisse der Signale der Patientenproben gegen die allgemeine Kontrolle berechnet. D.h. für das j-te Gen des n-ten Patienten ergab die Berechnung den Wert Gj,n = arcsinh(Scy5(j,n)) - arcsinh(Scy3(j,n)), wobei [SCy3(j,n), SCy5(j,n)] das zugehörige Signalpaar bezeichnet. War für einen Patienten ein Spot nicht auswertbar (z.B. Fleck auf dem gescannten Bild), so wurde der zugehörige Wert als nicht vorhanden ('missing value') gekennzeichnet.

### Statistischer Vergleich:

Für den Vergleich wurde der zweiseitige Zweistichproben Student-Test pro Gen verwendet. Beide Stichproben enthielten die Werte der Patientengruppen nichtinfektiöses MOV bzw. infektiöses MOV. Für die Auswahl der differenziert exprimierten Gene wurde der zugehörige p-Wert bewertet. Für die Gruppe der ausgewählten Gene war der zugehörige p-Wert kleiner als 0.05.

Die Höhe des Expressionsverhältnisses jedes Gens stellte das Kriterium für eine Sortierung der untersuchten Gene dar. Von Interesse waren die Gene, die zwischen den an einem nichtinfektiösen MOV erkrankten Patienten gegenüber den an infektiösen MOV erkrankten Patienten am meisten überexprimiert bzw. unterexprimiert wurden.

Aus Tabelle 2 ist ersichtlich, dass 721 Gene den Patientenprobe gefunden wurden, die in den Patienten mit infektiösem MOV gegenüber den Patienten mit nichtinfektiösem MOV signifikant überexprimiert waren. Weiterhin ist aus Tabelle 3 ersichtlich, dass 576 Gene der Patienten mit infektiösem MOV gegenüber den Patienten mit nichtinfektiösem MOV signifikant unterexprimiert waren. Aus den Ergebnissen wird deutlich, dass die in Tabelle 2 und Tabelle 3 aufgeführten Genaktivitäten zwischen den nichtinfektiösen Ursachen eines Multiorganversagen und den infektiösen Ursachen eines Multiorganversagen (unterscheiden. Somit stellen die aufgeführten Genaktivitäten Marker für eine Unterscheidung von nichtinfektiösen und infektiösen Ursachen eines Multiorganversagens dar.

**Tabelle 2: Signifikant gesteigerte Genaktivitäten in Proben von Patienten mit infektiösem MOV, im Vergleich zu den Genaktivitäten von Patienten mit nichtinfektiösem MOV**

| **GenBank Accession-Number** | **p-Wert** | **Mittlerer normalisierter und Standardabweichung transformierter** | | **Expressionswert Standardabweichung** | | **Sequenz-ID** |
|---|---|---|---|---|---|---|
| | | **Gruppe der Patienten mit nichtinfektiösem /MOV** | **Gruppe der Patienten mit infektiösem MOV** | **Gruppe der Patienten mit ID nichtinfektiösem /MOV** | **Gruppe der Patienten mit infektiösem MOV** | |
| N32857 | 0,00 | -2,99 | 0,20 | 1,42 | 2,78 | 1 |
| N32853 | 0,00 | -0,85 | 1,60 | 2,15 | 2,89 | 2 |
| N32495 | 0,00 | -2,38 | -0,56 | 1,37 | 0,40 | 3 |
| AI701077 | 0,01 | -0,33 | 1,46 | 0,17 | 3,08 | 4 |
| M87790 | 0,00 | 1,18 | 2,93 | 1,13 | 1,24 | 5 |
| AI559317 | 0,01 | 0,20 | 1,83 | 0,54 | 2,60 | 6 |
| N34897 | 0,00 | -2,60 | -1,05 | 1,61 | 0,54 | 7 |
| AA907084 | 0,02 | 0,53 | 1.94 | 0,49 | 2,58 | 8 |
| N45223 | 0,00 | -2,88 | -1,54 | 1,24 | 0,57 | 9 |
| H70430 | 0,03 | 0,12 | 1,42 | 0,70 | 2,73 | 10 |
| R59591 | 0,01 | -0,25 | 0,97 | 0,20 | 2,06 | 11 |
| N47688 | 0,00 | -2,47 | -1,27 | 0,94 | 0,44 | 12 |
| N52930 | 0,00 | -1,49 | -0,30 | 1,06 | 0,76 | 13 |
| XM_004256 | 0,00 | -3,10 | -1,94 | 0,62 | 1,43 | 14 |
| AJ010446 | 0,00 | -0,22 | 0,93 | 0,66 | 1,11 | 15 |
| N35225 | 0,00 | -2,81 | -1,75 | 1,21 | 0,52 | 16 |
| N50680 | 0,00 | -1,30 | -0,29 | 1,58 | 0,46 | 17 |
| BC018761 | 0,00 | 1,04 | 2,02 | 0,80 | 1,27 | 18 |
| XM_009475 | 0,00 | -2,54 | -1,58 | 0,83 | 0,91 | 19 |
| N53369 | 0,04 | -0.37 | 0,55 | 1,62 | 1,39 | 20 |
| AI420863 | 0,05 | -0,17 | 0,74 | 0,47 | 1,99 | 21 |
| N33423 | 0,05 | -0,32 | 0,58 | 1,64 | 1,51 | 22 |
| AA843281 | 0,05 | 0,27 | 1,15 | 0,54 | 1,88 | 23 |
| X64641 | 0,02 | 0,26 | 1,11 | 1,09 | 1,23 | 24 |
| N52545 | 0,00 | -1,10 | -0,28 | 1,02 | 0,55 | 25 |
| X57817 | 0.01 | 0,19 | 1,00 | 0,58 | 1,21 | 26 |
| N58236 | 0,00 | -0,68 | 0,14 | 0,85 | 0,56 | 27 |
| XM_056556 | 0,00 | -3,12 | -2,31 | 0,58 | 0,99 | 28 |
| N59170 | 0,01 | -0,22 | 0,59 | 1,35 | 0,74 | 29 |
| N58392 | 0,00 | -0,85 | -0,04 | 0,71 | 0,62 | 30 |
| N34672 | 0,02 | -0,54 | 0,26 | 1,74 | 0,39 | 31 |
| XM 015396 | 0,00 | -0,27 | 0,52 | 0,68 | 0,81 | 32 |
| X05875 | 0,01 | -2,86 | -2,09 | 0,55 | 1,15 | 33 |
| N48715 | 0,00 | -1,12 | -0,36 | 0,70 | 0,61 | 34 |
| N90140 | 0,05 | -0,44 | 0,32 | 0,29 | 1,71 | 35 |
| NM 002415 | 0,00 | -1,66 | -0,90 | 0,63 | 0,56 | 36 |
| AI890242 | 0,00 | -0,14 | 0,59 | 0,25 | 0,72 | 37 |
| AI589096 | 0,00 | -0,39 | 0,32 | 0,56 | 0,54 | 38 |
| NM_001911 | 0,04 | -2,61 | -1,91 | 0,77 | 1,44 | 39 |
| N39242 | 0,05 | -0,56 | 0,12 | 1,75 | 0,52 | 40 |
| N35493 | 0,04 | -0,45 | 0,23 | 1,69 | 0,46 | 41 |
| AI271764 | 0,00 | -0,66 | -0,02 | 0,70 | 0,62 | 42 |
| NM_006936 | 0,00 | -2,00 | -1,37 | 0,46 | 0,55 | 43 |
| NM_005225 | 0,00 | -0,90 | -0,26 | 0,58 | 0,53 | 44 |
| R98960 | 0,04 | -0,37 | 0,26 | 1,41 | 0,70 | 45 |
| NM_000714.3 | 0,00 | 0,48 | 1,11 | 0,44 | 0,88 | 46 |
| N48180 | 0,01 | -1,08 | -0,45 | 1,05 | 0,45 | 47 |
| NM_002295 | 0,02 | -3,17 | -2,56 | 0,44 | 1,09 | 48 |
| AI697365 | 0,01 | 0,62 | 1,22 | 0,82 | 0,67 | 49 |
| NM_001404 | 0,00 | -2,56 | -1,96 | 0,38 | 0,86 | 50 |
| NM_176800.1 | 0,00 | -0,31 | 0,29 | 0,51 | 0,42 | 51 |
| XM_027885 | 0,03 | -3,23 | -2,63 | 0,37 | 1,17 | 52 |
| NM_006597.3 | 0,00 | -2,42 | -1,84 | 0,50 | 0,79 | 53 |
| NM_002211 | 0,00 | -1,59 | -1,02 | 0,68 | 0,54 | 54 |
| NM_001570 | 0,00 | -0,55 | 0,03 | 0,53 | 0,49 | 55 |
| AI888606 | 0,03 | -0,16 | 0,40 | 0,44 | 1,11 | 56 |
| NM_006636.2 | 0,04 | -3,49 | -2,93 | 0,53 | 1,16 | 57 |
| AA458827 | 0,00 | 0,15 | 0,71 | 0,33 | 0,60 | 58 |
| AA398757 | 0,01 | 0,11 | 0,67 | 0,57 | 0,81 | 59 |
| NM_000814.2 | 0,00 | -0,07 | 0,49 | 0,48 | 0,75 | 60 |
| NM_000963 | 0,00 | -0,41 | 0,15 | 0,86 | 0,33 | 61 |
| AI913322 | 0,02 | -0,68 | -0,14 | 0,68 | 0,92 | 62 |
| N20922 | 0,04 | -0,72 | -0,17 | 1,27 | 0,51 | 63 |
| R49085 | | 0,00 0,02 | 0,57 | 0,64 | 0,56 | 64 |
| N54935 | 0,01 | -0,74 | -0,19 | 0,97 | 0,38 | 65 |
| XM_027358 | 0,01 | -1,49 | -0,95 | 0,75 | 0,59 | 66 |
| NM_031200 | 0,00 | 0,11 | 0,65 | 0,57 | 0,57 | 67 |
| AA805531 | 0,00 | -0,07 | 0,47 | 0,33 | 0,53 | 68 |
| NM_000194 | 0,04 | -2,64 | -2,12 | 0,70 | 1,02 | 69 |
| AI623567 | 0,01 | 0,39 | 0,92 | 0,59 | 0,73 | 70 |
| N64495 | 0,00 | -0,49 | 0,02 | 0,63 | 0,37 | 71 |
| NM_002156 | 0,01 | -2,26 | -1,75 | 0,59 | 0,70 | 72 |
| NM_012068 | 0,00 | -1,40 | -0,89 | 0,54 | 0,40 | 73 |
| R43722 | 0,02 | -0,45 | 0,05 | 0,65 | 0,80 | 74 |
| NM_001686 | 0,03 | -2,63 | -2,13 | 0,32 | 0,93 | 75 |
| NM_002969 | 0,00 | -0,92 | -0,42 | 0,46 | 0,53 | 76 |
| NM_003295 | 0,04 | -2,72 | -2,24 | 0,45 | 0,98 | 77 |
| XM 039372 | 0,02 | -2,43 | -1,95 | 0,26 | 0,92 | 78 |
| AA731679 | 0,02 | 0,17 | 0,65 | 0,79 | 0,61 | 79 |
| AA620762 | 0,00 | -0,04 | 0,44 | 0,21 | 0,50 | 80 |
| AI499889 | 0,01 | -0,01 | 0,47 | 0,67 | 0,64 | 81 |
| N33530 | 0,00 | -0,30 | 0,18 | 0,70 | 0,31 | 82 |
| NM_002033 | 0,00 | -1,92 | -1,44 | 0,39 | 0,63 | 83 |
| AA436651 | 0,00 | -0,26 | 0,21 | 0,54 | 0,26 | 84 |
| NM_001540 | 0,00 | -1,42 | -0,95 | 0,42 | 0,54 | 85 |
| NM_004257 | 0,00 | -0,85 | -0,38 | 0,33 | 0,25 | 86 |
| NM_014280.1 | 0,00 | -1,45 | -0,98 | 0,58 | 0,47 | 87 |
| NM_000930.2 | 0,00 | -1,30 | -0,83 | 0,64 | 0,51 | 88 |
| XM 002101 | 0,00 | -0,63 | -0,17 | 0,63 | 0,27 | 89 |
| AI733269 | 0,00 | -0,18 | 0,29 | 0,45 | 0,36 | 90 |
| NM_001168 | 0,02 | -2,14 | -1,67 | 0,61 | 0,77 | 91 |
| XM_052636 | 0,00 | -1,51 | -1,04 | 0,35 | 0,48 | 92 |
| AI689318 | 0,00 | -1,00 | -0,54 | 0,55 | 0,46 | 93 |
| NM_001212 | 0,01 | -1,65 | -1,19 | 0,56 | 0,64 | 94 |
| R37251 | 0,00 | 0,61 | 1,06 | 0,39 | 0,63 | 95 |
| NM_001166 | 0,00 | -0,76 | -0,31 | 0,53 | 0,41 | 96 |
| XM 056798 | 0,01 | -1,34 | -0,89 | 0,66 | 0,51 | 97 |
| NM_005052 | 0,01 | 0,41 | 0,86 | 0,37 | 0,67 | 98 |
| NM_003379 | 0,00 | -1,45 | -1,00 | 0,41 | 0,51 | 99 |
| XM 048068 | 0,00 | -0,37 | 0,08 | 0,43 | 0,42 | 100 |
| NM_000577 | 0,01 | 0,45 | 0,90 | 0,32 | 0,67 | 101 |
| NM_001101 | 0,00 | -0,69 | -0,25 | 0,43 | 0,58 | 102 |
| D31890 | 0,01 | -1,79 | -1,36 | 0,56 | 0,55 | 103 |
| N49976 | 0,03 | -0,26 | 0,17 | 0,90 | 0,50 | 104 |
| XM 008679 | 0,01 | -0,85 | -0,41 | 0,57 | 0,56 | 105 |
| N33187 | 0,01 | -0,06 | 0,38 | 0,52 | 0,54 | 106 |
| R42782 | 0,00 | -0,09 | 0,34 | 0,36 | 0,45 | 107 |
| N49751 | 0,01 | 0,71 | 1,14 | 0,48 | 0,64 | 108 |
| AI910456 | 0,04 | -1,12 | -0,69 | 0,73 | 0,75 | 109 |
| NM 001569 | 0,00 | -1,10 | -0,67 | 0,38 | 0,46 | 110 |
| H90322 | 0,00 | 0,05 | 0,48 | 0,27 | 0,51 | 111 |
| AI926659 | 0,00 | 0,05 | 0,48 | 0,37 | 0,44 | 112 |
| XM 047499 | 0,01 | -1,29 | -0,86 | 0,46 | 0,67 | 113 |
| AA437224 | 0,00 | -0,71 | -0,28 | 0,46 | 0,24 | 114 |
| NM_021798 | 0,00 | -0,32 | 0,11 | 0,44 | 0,36 | 115 |
| NM 000584 | 0,02 | -1,82 | -1,40 | 0,61 | 0,64 | 116 |
| AA452122 | 0,00 | -0,40 | 0,02 | 0,60 | 0,41 | 117 |
| NM_002189 | 0,01 | 0,10 | 0,52 | 0,48 | 0,57 | 118 |
| AA001367 | 0,00 | -0,13 | 0,29 | 0,37 | 0,57 | 119 |
| AI129679 | 0,00 | -1,27 | -0,85 | 0,31 | 0,37 | 120 |
| D26599 | 0,01 | -1,90 | -1,48 | 0,50 | 0,58 | 121 |
| NM_170665.2 | 0,00 | -1,19 | -0,78 | 0,49 | 0,45 | 122 |
| NM_006419 | 0,00 | -0,16 | 0,25 | 0,39 | 0,51 | 123 |
| W85706 | 0,00 | -1,07 | -0,66 | 0,30 | 0,37 | 124 |
| AA897528 | 0,00 | -0,50 | -0,09 | 0,65 | 0,30 | 125 |
| NM_003358 | 0,04 | 0,56 | 0,97 | 0,50 | 0,82 | 126 |
| N35251 | 0,00 | -0,18 | 0,22 | 0,52 | 0,41 | 127 |
| NM_004863 | 0,00 | -0,63 | -0,22 | 0,37 | 0,48 | 128 |
| NM_001950 | 0,00 | -0,82 | -0,41 | 0,40 | 0,33 | 129 |
| NM_006260 | 0,03 | -0,63 | -0,22 | 0,61 | 0,67 | 130 |
| NM_170708 | 0,03 | -1,52 | -1,12 | 0,54 | 0,63 | 131 |
| N63024 | 0,01 | 0,64 | 1,04 | 0,43 | 0,56 | 132 |
| NM_017595 | 0,00 | -0,85 | -0,45 | 0,36 | 0,33 | 133 |
| AI364529 | 0,02 | -0,97 | -0,57 | 0,59 | 0,59 | 134 |
| NM_013432 | 0,00 | -0,30 | 0,10 | 0,31 | 0,28 | 135 |
| NM_006736.2 | 0,00 | -0,56 | -0,16 | 0,24 | 0,37 | 136 |
| NM_002128 | 0,02 | -1,84 | -1,44 | 0,40 | 0,69 | 137 |
| AA441793 | 0,00 | -0,70 | -0,31 | 0,45 | 0,33 | 138 |
| N76019 | 0,00 | -0,27 | 0,13 | 0,35 | 0,30 | 139 |
| XM 048665 | 0,00 | -0,28 | 0,11 | 0,38 | 0,35 | 140 |
| NM_003467 | 0,01 | -1,80 | -1,41 | 0,32 | 0,62 | 141 |
| N59330 | 0,01 | -0,21 | 0,19 | 0,56 | 0,50 | 142 |
| NM_004672 | 0,00 | -0,08 | 0,32 | 0,44 | 0,26 | 143 |
| AA426021 | 0,01 | 0,06 | 0,45 | 0,28 | 0.61 | 144 |
| XM_008608 | 0,00 | -0,60 | -0,21 | 0,54 | 0,34 | 145 |
| H44908 | 0,00 | -0,55 | -0,16 | 0,42 | 0,33 | 146 |
| AA699412 | 0,00 | -0,47 | -0,08 | 0,48 | 0,35 | 147 |
| AI572080 | 0,01 | 0,28 | 0,67 | 0,41 | 0,52 | 148 |
| NM_012072 | 0,02 | -1,86 | -1,47 | 0,47 | 0,63 | 149 |
| XM 035638 | 0,04 | -1,96 | -1,57 | 0,40 | 0,80 | 150 |
| BC001604 | 0,00 | -1,13 | -0,74 | 0,40 | 0,33 | 151 |
| AA481282 | 0,00 | -0,11 | 0,27 | 0,54 | 0,40 | 152 |
| NM_003376 | 0,01 | -1,17 | -0,78 | 0,54 | 0,42 | 153 |
| H11661 | 0,00 | -0,07 | 0,32 | 0,25 | 0,37 | 154 |
| AI435179 | 0,01 | -0,08 | 0,30 | 0,68 | 0,37 | 155 |
| XM_006800 | 0,01 | 0,19 | 0,57 | 0,38 | 0,55 | 156 |
| NM_000397.2 | 0,00 | -0,56 | -0,17 | 0,37 | 0,28 | 157 |
| AA424023 | 0,02 | 0,01 | 0,39 | 0,43 | 0,63 | 158 |
| XM 012949 | 0,02 | -1,81 | -1,43 | 0,45 | 0,64 | 159 |
| W84866 | 0,00 | 0,14 | 0,52 | 0,44 | 0,45 | 160 |
| N62672 | 0,01 | -0,21 | 0,17 | 0,60 | 0,44 | 161 |
| NM_001530 | 0,01 | -0,16 | 0,21 | 0,25 | 0,62 | 162 |
| NM_002157.1 | 0,03 | -2,21 | -1,83 | 0,43 | 0,71 | 163 |
| NM_003258 | 0,02 | -1,80 | -1,43 | 0,68 | 0,46 | 164 |
| AI863135 | 0,04 | 0,87 | 1,25 | 0,40 | 0,76 | 165 |
| NM_004083 | 0,01 | -0,95 | -0,58 | 0,46 | 0,47 | 166 |
| H06194 | 0,00 | -0,92 | -0,54 | 0,45 | 0,30 | 167 |
| XM_047570 | 0,03 | -1,61 | -1,24 | 0,41 | 0,68 | 168 |
| D26598 | 0,01 | -1,23 | -0,86 | 0,27 | 0,57 | 169 |
| R44955 | 0,01 | -0,08 | 0,29 | 0,55 | 0,49 | 170 |
| NM_012297 | 0,02 | -1,60 | -1,22 | 0,48 | 0,59 | 171 |
| T84080 | 0,02 | 0,13 | 0,49 | 0,57 | 0,52 | 172 |
| H52810 | 0,00 | 0,13 | 0,50 | 0,33 | 0,44 | 173 |
| XM_055188 | 0,04 | 0,94 | 1,30 | 0,36 | 0,75 | 174 |
| AI184987 | 0,01 | 0,19 | 0,56 | 0,51 | 0,50 | 175 |
| AI733177 | 0,02 | 0,54 | 0,90 | 0,41 | 0,63 | 176 |
| NM_006016 | 0,02 | -1,15 | -0,78 | 0,44 | 0,60 | 177 |
| XM_006867 | 0,02 | 0,09 | 0,46 | 0,33 | 0,62 | 178 |
| NM_004475.1 | 0,02 | 0,95 | 1,32 | 0,40 | 0,60 | 179 |
| AA485242 | 0,03 | 0,34 0,70 | | 0,49 | 0.62 | 180 |
| NM_003300 | 0,01 | -1,49 | -1,13 | 0,31 | 0,54 | 181 |
| NM_032957 | 0,00 | -0.88 | -0,52 | 0,32 | 0,39 | 182 |
| XM_033862 | 0,00 | -0,01 | 0,35 | 0,29 | 0,36 | 183 |
| W80385 | 0,01 | 0,10 | 0,46 | 0,36 | 0,52 | 184 |
| H99099 | 0,01 | -0,04 | 0,32 | 0,37 | 0,52 | 185 |
| N67859 | 0,00 | -0.77 | -0,41 | 0,34 | 0,39 | 186 |
| NM_001013 | 0,04 | -1,88 | -1,52 | 0,44 | 0,68 | 187 |
| NM_006641 | 0,02 | -0,36 | 0,00 | 0,69 | 0,35 | 188 |
| N70546 | 0,00 | -0,11 | 0,25 | 0,38 | 0,40 | 189 |
| XM_015278 | 0,00 | -0,36 | -0,01 | 0,33 | 0,42 | 190 |
| AI932670 | 0,00 | -0,15 | 0,20 | 0,36 | 0,43 | 191 |
| NM_175617 | 0,00 | -0,11 | 0,25 | 0,29 | 0,26 | 192 |
| NM_004377.2 | 0,02 | -0,88 | -0,53 | 0,52 | 0,50 | 193 |
| NM_003153 | 0,00 | -0,39 | -0,04 | 0,30 | 0,48 | 194 |
| AI910804 | 0,03 | -0,65 | -0,30 | 0,51 | 0,57 | 195 |
| AI221860 | 0,00 | -0,30 | 0,05 | 0,17 | 0,43 | 196 |
| AI866414 | 0,00 | -0,37 | -0,02 | 0,33 | 0,27 | 197 |
| BC020968 | 0,03 | -1,77 | -1,42 | 0,36 | 0,65 | 198 |
| AA484213 | 0,05 | -0,49 | -0,14 | 0,90 | 0,27 | 199 |
| XM_003593 | 0,00 | -0,52 | -0,17 | 0,44 | 0,27 | 200 |
| XM_008738 | 0,02 | -1,46 | -1,11 | 0,54 | 0,49 | 201 |
| NM 032964 | 0,00 | -0,48 | -0,13 | 0,41 | 0,20 | 202 |
| NM_001455 | 0,00 | 0,26 | 0,61 | 0,42 | 0,41 | 203 |
| NM 002994 | 0,00 | -0,61 | -0,26 | 0,35 | 0,43 | 204 |
| NM_004222 | 0,00 | -1,44 | -1,10 | 0,30 | 0,45 | 205 |
| H48923 | 0,00 | -0.59 | -0,25 | 0,35 | 0,39 | 206 |
| T47430 | 0,05 | 0,41 | 0,75 | 0,38 | 0,71 | 207 |
| NM_032963 | 0,00 | -0,45 | -0,11 | 0,52 | 0,22 | 208 |
| XM_045933 | 0,00 | 0.22 | 0,56 | 0,23 | 0,41 | 209 |
| T99746 | 0,03 | 0,26 | 0,60 | 0,49 | 0,52 | 210 |
| XM_012039 | 0,01 | -1,44 | -1,10 | 0,44 | 0,43 | 211 |
| NM_004740 | 0,00 | -0.46 | -0,12 | 0,29 | 0,24 | 212 |
| NM 001681.2 | 0,05 | -1,39 | -1,05 | 0,61 | 0,60 | 213 |
| AI027259 | 0,00 | -0,40 | -0,06 | 0,49 | 0,28 | 214 |
| AA431552 | 0,00 | -0,63 | -0,30 | 0,41 | 0,32 | 215 |
| NM_000029 | 0,00 | 0,30 | 0,63 | 0,32 | 0,42 | 216 |
| XM_041847 | 0,05 | -1,02 | -0,68 | 0,69 | 0,51 | 217 |
| NM_005920 | 0,00 | -0,90 | -0,56 | 0,28 | 0,33 | 218 |
| NM_002394 | 0,01 | -1,03 | -0,69 | 0,49 | 0,39 | 219 |
| AI093704 | 0,01 | -0,32 | 0,02 | 0,35 | 0,47 | 220 |
| XM_043359 | 0,01 | 0,21 | 0,55 | 0,36 | 0,51 | 221 |
| H48445 | 0,01 | 0,28 | 0,61 | 0,38 | 0,53 | 222 |
| XM_015815 | 0,02 | -1,13 | -0,80 | 0,52 | 0,50 | 223 |
| NM_001774 | 0,00 | -0,07 | 0,27 | 0,31 | 0,42 | 224 |
| AI937053 | 0,00 | -0,42 | -0,09 | 0,39 | 0,26 | 225 |
| AA493719 | 0,01 | -0,61 | -0,28 | 0,52 | 0,35 | 226 |
| NM_002996 | 0,01 | 0,19 | 0,51 | 0,33 | 0,44 | 227 |
| AI025039 | 0,01 | 0,16 | 0,49 | 0,31 | 0,47 | 228 |
| NM_139049 | 0,02 | -0,52 | -0,19 | 0,60 | 0,34 | 229 |
| NM_006238.2 | 0,00 | -0,29 | 0,04 | 0,21 | 0,26 | 230 |
| XM_031456 | 0,00 | -0,67 | -0,35 | 0,30 | 0,30 | 231 |
| AA455096 | 0,00 | -0,29 | 0,03 | 0,29 | 0,32 | 232 |
| XM_047675 | 0,03 | 0,36 | 0,68 | 0,26 | 0,65 | 233 |
| AI809252 | 0,00 | -0,14 | 0,18 | 0,36 | 0,34 | 234 |
| NM_139047 | 0,00 | -0,45 | -0,13 | 0,47 | 0,31 | 235 |
| AI760793 | 0,01 | -0,47 | -0,15 | 0,47 | 0,33 | 236 |
| NM_000204 | 0,00 | -0,03 | 0,29 | 0,25 | 0,40 | 237 |
| AI860121 | 0,01 | 0,55 | 0,87 | 0.37 | 0,48 | 238 |
| H50222 | 0,00 | -0,13 | 0,19 | 0,19 | 0,34 | 239 |
| XM_041101 | 0,02 | -1,06 | -0,74 | 0,34 | 0,56 | 240 |
| XM_035854 | 0,01 | 0,09 | 0,41 | 0,47 | 0,44 | 241 |
| AA043903 | 0,01 | -0,73 | -0,41 | 0,52 | 0,31 | 242 |
| R40406 | 0,03 | -0,89 | -0,58 | 0,48 | 0,52 | 243 |
| N98510 | 0,04 | -1,22 | -0,90 | 0,51 | 0,56 | 244 |
| H05449 | 0,03 | -0,15 | 0,16 | 0,52 | 0,50 | 245 |
| AI567338 | 0,01 | -0,11 | 0,20 | 0,43 | 0,39 | 246 |
| NM 000308.1 | 0,00 | -0,19 | 0,13 | 0,29 | 0,42 | 247 |
| R40880 | 0,00 | -0,21 | 0,11 | 0,40 | 0,34 | 248 |
| H52284 | 0,00 | -0,26 | 0,05 | 0,41 | 0,27 | 249 |
| NM_030662 | 0,00 | -0,47 | -0,16 | 0,22 | 0,27 | 250 |
| NM_032965 | 0,02 | 0,52 | 0,83 | 0,41 | 0,47 | 251 |
| NM_004322 | 0,00 | -0,34 | -0,03 | 0,32 | 0,27 | 252 |
| XM_002762 | 0,00 | -0,52 | -0,21 | 0,22 | 0,25 | 253 |
| AI679230 | 0,00 | -0,40 | -0,09 | 0,43 | 0,33 | 254 |
| AI368670 | 0,00 | -0,23 | 0,08 | 0,30 | 0,31 | 255 |
| NM_006415 | 0,01 | -0,71 | -0,40 | 0,45 | 0,33 | 256 |
| NM_004379 | 0,00 | -0,54 | -0,23 | 0,24 | 0,24 | 257 |
| NM_002974 | 0,02 | 0,02 | 0,33 | 0,49 | 0,38 | 258 |
| AI914729 | 0,02 | -0,11 | 0,20 | 0,51 | 0,40 | 259 |
| NM_032989 | 0,00 | -0,17 | 0,14 | 0,23 | 0,28 | 260 |
| AI799645 | 0,04 | 0,14 | 0,45 | 0,39 | 0,61 | 261 |
| AA436553 | 0,01 | -0,33 | -0,03 | 0,59 | 0,24 | 262 |
| NM_033015 | 0,00 | -0,37 | -0,06 | 0,32 | 0,25 | 263 |
| XM_002224 | 0,01 | -0,26 | 0,04 | 0,57 | 0,28 | 264 |
| AI708030 | 0,00 | 0,11 | 0,41 | 0,37 | 0,32 | 265 |
| A1041544 | 0,00 | -0,28 | 0,02 | 0,28 | 0,27 | 266 |
| NM_005801 | 0,03 | -1,39 | -1,09 | 0,36 | 0,54 | 267 |
| NM_022559 | 0,00 | -0,63 | -0,33 | 0,42 | 0,24 | 268 |
| XM_043864 | 0,00 | -0,40 | -0,10 | 0,32 | 0,31 | 269 |
| NM_003840 | 0,01 | -0,42 | -0,12 | 0,52 | 0,25 | 270 |
| AI565083 | 0,00 | -0,28 | 0,02 | 0,32 | 0,24 | 271 |
| R91168 | 0,01 | 0,15 | 0,45 | 0,36 | 0,40 | 272 |
| AI799787 | 0,01 | 0,12 | 0,41 | 0,34 | 0,38 | 273 |
| AI652564 | 0,00 | -0,91 | -0,61 | 0,31 | 0,30 | 274 |
| H05310 | 0,00 | 0,02 | 0,32 | 0,30 | 0,29 | 275 |
| AA708806 | 0,00 | -0,22 | 0,08 | 0,24 | 0,26 | 276 |
| H74205 | 0,03 | 0,07 | 0,37 | 0,42 | 0,49 | 277 |
| NM_000061 | 0,03 | -1,59 | -1,30 | 0,40 | 0,53 | 278 |
| NM_003110.3 | 0,00 | -0,29 | 0,00 | 0,38 | 0,27 | 279 |
| AA625887 | 0,00 | -0,30 | -0,01 | 0,22 | 0,23 | 280 |
| H41124 | 0,03 | -0,37 | -0,08 | 0,39 | 0,48 | 281 |
| AI769514 | 0,02 | -0,58 | -0,28 | 0,56 | 0,35 | 282 |
| XM_036107 | 0,03 | -0.27 | 0,02 | 0,27 | 0,53 | 283 |
| R52679 | 0,01 | -1,03 | -0,74 | 0,36 | 0,35 | 284 |
| AI217811 | 0,04 | 0,02 | 0,31 | 0,31 | 0,59 | 285 |
| NM_004168 | 0,02 | -0,63 | -0,34 | 0,42 | 0,44 | 286 |
| AI933607 | 0,03 | -0,24 | 0,05 | 0,32 | 0,55 | 287 |
| NM_007052.3 | 0,03 | -0,24 | 0,05 | 0,61 | 0,29 | 288 |
| AI799137 | 0,02 | -0,42 | -0,12 | 0,52 | 0,37 | 289 |
| NM_002720 | 0,00 | -0,64 | -0,35 | 0,28 | 0,34 | 290 |
| R26635 | 0,04 | -0,16 | 0,13 | 0,32 | 0,57 | 291 |
| AI625594 | 0,00 | -0,01 | 0,28 | 0,29 | 0,31 | 292 |
| NM_001562 | 0,00 | -0,42 | -0,13 | 0,22 | 0,27 | 293 |
| W93717 | 0,05 | 0,11 | 0,40 | 0,64 | 0,40 | 294 |
| NM_002521.1 | 0,01 | -0,10 | 0,19 | 0,44 | 0,29 | 295 |
| R42543 | 0,05 | 0,26 | 0,55 | 0,45 | 0,54 | 296 |
| AI302949 | 0,00 | -0,13 | 0,16 | 0,21 | 0,23 | 297 |
| H54279 | 0,00 | -0,01 | 0,27 | 0,29 | 0,32 | 298 |
| AI219513 | 0,00 | -0,47 | -0,19 | 0,41 | 0,27 | 299 |
| N68173 | 0,00 | -0,11 | 0,18 | 0,26 | 0,38 | 300 |
| AA496235 | 0,00 | -0,38 | -0,09 | 0,41 | 0.28 | 301 |
| AI742529 | 0,03 | 0,39 | 0,67 | 0,33 | 0,51 | 302 |
| H79534 | 0,01 | -0,50 | -0,21 | 0,47 | 0,32 | 303 |
| AA002267 | 0,03 | -0,04 | 0,25 | 0,36 | 0,47 | 304 |
| H52638 | 0,00 | -0,38 | -0,10 | 0,40 | 0,28 | 305 |
| N70324 | 0,01 | 0,07 | 0,35 | 0,47 | 0,33 | 306 |
| NM_003805 | 0,02 | -0,83 | -0,55 | 0,47 | 0,36 | 307 |
| N59766 | 0,03 | -0,04 | 0,24 | 0,41 | 0,43 | 308 |
| XM_034770 | 0,04 | -1,26 | -0,98 | 0,44 | 0,45 | 309 |
| AI538438 | 0,01 | 0,06 | 0,34 | 0,43 | 0,31 | 310 |
| AI250800 | 0,00 | 0,05 | 0,32 | 0,23 | 0,29 | 311 |
| AA845475 | 0,00 | -0,24 | 0,04 | 0,39 | 0,25 | 312 |
| AI700169 | 0,00 | -0,23 | 0,05 | 0,33 | 0,30 | 313 |
| NM_003639) | 0,00 | -0,37 | -0,09 | 0,26 | 0,31 | 314 |
| AI125864 | 0,03 | -0,27 | 0,01 | 0,60 | 0,24 | 315 |
| NM 000757 | 0,03 | 0,12 | 0,40 | 0,38 | 0,48 | 316 |
| NM_006216 | 0,03 | -0,12 | 0,16 | 0,45 | 0,42 | 317 |
| A1077481 | 0,04 | 0,21 | 0,49 | 0,41 | 0,48 | 318 |
| AI149647 | 0,03 | -0,13 | 0,15 | 0,43 | 0,46 | 319 |
| XM_030906 | 0,01 | 0,18 | 0,45 | 0,28 | 0,39 | 320 |
| NM_004834 | 0,00 | -0,68 | -0,41 | 0,27 | 0,35 | 321 |
| XM_031287 | 0,01 | 0,11 | 0,38 | 0,27 | 0,41 | 322 |
| AI923251 | 0,00 | -0,25 | 0,02 | 0,20 | 0,24 | 323 |
| AI203697 | 0,00 | -0,22 | 0,05 | 0,31 | 0,18 | 324 |
| AA621192 | 0,02 | 0,04 | 0,31 | 0,34 | 0,45 | 325 |
| XM_008450 | 0,02 | -0,24 | 0,03 | 0,51 | 0,29 | 326 |
| AI540674 | 0,00 | -0,76 | -0,49 | 0,25 | 0,34 | 327 |
| AA514237 | 0,03 | 0,35 | 0,62 | 0,31 | 0,47 | 328 |
| AI348271 | 0,01 | -0,11 | 0,16 | 0,45 | 0,26 | 329 |
| NM_000684.1 | 0,02 | 0,41 | 0,68 | 0,29 | 0,44 | 330 |
| NM 001951 | 0,03 | -0,67 | -0.40 | 0,50 | 0,40 | 331 |
| N55249 | 0,01 | -0,42 | -0,15 | 0,45 | 0,31 | 332 |
| AI150732 | 0,01 | -0,20 | 0,07 | 0,34 | 0,34 | 333 |
| AI147315 | 0,03 | 0,35 | 0,62 | 0,38 | 0,45 | 334 |
| NM_003010 | 0,00 | -0,46 | -0,20 | 0,34 | 0,25 | 335 |
| AA460460 | 0,01 | -0,22 | 0,05 | 0,47 | 0,28 | 336 |
| AI651337 | 0,01 | -0,49 | -0,22 | 0,41 | 0,31 | 337 |
| AA971087 | 0,01 | -0,19 | 0,08 | 0,42 | 0,25 | 338 |
| NM_003811 | 0,03 | -1,09 | -0,82 | 0,50 | 0,37 | 339 |
| XM_053519 | 0,01 | -0,30 | -0,04 | 0,26 | 0,39 | 340 |
| NM_001609.1 | 0,00 | -0,24 | 0,03 | 0,29 | 0,29 | 341 |
| AA463423 | 0,00 | -0,17 | 0,09 | 0,22 | 0,35 | 342 |
| AA648848 | 0,02 | 0,04 | 0,30 | 0,35 | 0,42 | 343 |
| AI141692 | 0,05 | -0,12 | 0,14 | 0,67 | 0,22 | 344 |
| R79239 | 0,04 | 0,06 | 0,33 | 0,50 | 0,42 | 345 |
| AI298171 | 0,00 | -0,28 | -0,01 | 0,17 | 0,21 | 346 |
| H17432 | 0,03 | -0,29 | -0,03 | 0,57 | 0,23 | 347 |
| NM_004635 | 0,05 | -1,36 | -1,10 | 0,31 | 0,51 | 348 |
| NM_005409 | 0,02 | 0,14 | 0,40 | 0,20 | 0,50 | 349 |
| A1452845 | 0,03 | 0,23 | 0,49 | 0,37 | 0,43 | 350 |
| AI222914 | 0,00 | -0,03 | 0,23 | 0,29 | 0,24 | 351 |
| AI885492 | 0,00 | -0,06 | 0,20 | 0,34 | 0,20 | 352 |
| NM 002953 | 0,01 | -0,61 | -0,35 | 0,24 | 0,41 | 353 |
| AI201175 | 0,01 | 0,25 | 0,51 | 0,30 | 0,33 | 354 |
| NM_001735 | 0,02 | -0,45 | -0,20 | 0,47 | 0,29 | 355 |
| D78151 | 0,02 | -0,70 | -0,44 | 0,42 | 0,34 | 356 |
| NM_006712 | 0,00 | -0,20 | 0,06 | 0,36 | 0,24 | 357 |
| AF004429 | 0,00 | -0,63 | -0,37 | 0,29 | 0,29 | 358 |
| NM_031409 | 0,03 | 0,19 | 0,44 | 0,32 | 0,45 | 359 |
| AI742287 | 0,01 | -0,30 | -0,04 | 0,41 | 0,27 | 360 |
| BC015542 | 0,02 | -0,42 | -0,16 | 0,34 | 0,42 | 361 |
| AI685923 | 0,00 | -0,43 | -0,18 | 0,32 | 0,22 | 362 |
| NM_002218.1 | 0,01 | -0,55 | -0,29 | 0,34 | 0,28 | 363 |
| XM_003913 | 0,00 | -0,05 | 0,20 | 0,29 | 0,29 | 364 |
| N53480 | 0,02 | -0,64 | -0,39 | 0,40 | 0,38 | 365 |
| XM_048511 | 0,00 | -0.35 | -0,10 | 0,37 | 0,25 | 366 |
| R06710 | 0,02 | 0,05 | 0,30 | 0,39 | 0,35 | 367 |
| AI694720 | 0,01 | 0,29 | 0,54 | 0,28 | 0,34 | 368 |
| AI910988 | 0,00 | 0,07 | 0,32 | 0,23 | 0,33 | 369 |
| AA411624 | 0,02 | -0,52 | -0,27 | 0,33 | 0,38 | 370 |
| BC024270 | 0,00 | -0,43 | -0,18 | 0,32 | 0,27 | 371 |
| T90460 | 0,01 | -0,39 | -0,14 | 0,48 | 0,17 | 372 |
| NM_004850 | 0,02 | -0,92 | -0,67 | 0,41 | 0,32 | 373 |
| AA044390 | 0,03 | -0,01 | 0,24 | 0,26 | 0,45 | 374 |
| NM_005347.2 | 0,00 | -0,26 | -0,01 | 0,21 | 0,23 | 375 |
| XM_027216 | 0,03 | -0,68 | -0,43 | 0,42 | 0,39 | 376 |
| H53259 | 0,04 | 0,45 | 0,70 | 0,35 | 0,45 | 377 |
| R26717 | 0,02 | -0,02 | 0,22 | 0,40 | 0,35 | 378 |
| AI912970 | 0,02 | 0,19 | 0,44 | 0,36 | 0,35 | 379 |
| XM_001687 | 0,04 | -0,91 | -0,66 | 0,44 | 0,40 | 380 |
| NM_000565 | 0,04 | -0,67 | -0,43 | 0,27 | 0,50 | 381 |
| AI374990 | 0,01 | -0,14 | 0,10 | 0,31 | 0,35 | 382 |
| N22563 | 0,02 | 0,12 | 0,37 | 0,33 | 0,39 | 383 |
| AI764969 | 0,03 | -0,18 | 0,07 | 0,53 | 0,25 | 384 |
| AA417950 | 0,02 | -0,34 | -0,09 | 0,48 | 0,26 | 385 |
| H15431 | 0,03 | -0,39 | -0,14 | 0,51 | 0,30 | 386 |
| AI147997 | 0,02 | 0,11 | 0,35 | 0,24 | 0,45 | 387 |
| AI378142 | 0,03 | -0,10 | 0,14 | 0,25 | 0,42 | 388 |
| AA528101 | 0,00 | -0,43 | -0,19 | 0,32 | 0,21 | 389 |
| T83761 | 0,04 | -0,41 | -0,16 | 0,36 | 0,46 | 390 |
| XM_046674 | 0,04 | -0,80 | -0,56 | 0,59 | 0,21 | 391 |
| AI925556 | 0,00 | -0,53 | -0,29 | 0,24 | 0,21 | 392 |
| N50785 | 0,03 | 0,25 | 0,49 | 0,29 | 0,43 | 393 |
| AI739085 | 0,04 | -0,41 | -0,17 | 0,41 | 0,39 | 394 |
| AA885052 | 0,02 | -0,13 | 0,11 | 0,42 | 0,28 | 395 |
| R45218 | 0,01 | 0,17 | 0,41 | 0,25 | 0,37 | 396 |
| N71365 | 0,00 | -0,39 | -0,15 | 0,22 | 0,33 | 397 |
| AI590053 | 0,00 | -0,38 | -0,14 | 0,19 | 0,16 | 398 |
| NM_013229 | 0,00 | 0,02 | 0,26 | 0,22 | 0,26 | 399 |
| NM_001196 | 0,02 | -0,53 | -0,30 | 0,27 | 0,39 | 400 |
| R94509 | 0,01 | 0,07 | 0,31 | 0,29 | 0,29 | 401 |
| AA282936 | 0,02 | 0,16 | 0,39 | 0,41 | 0,33 | 402 |
| NM_003824 | 0,01 | -0,74 | -0,50 | 0,26 | 0,37 | 403 |
| T65296 | 0,01 | 0,00 | 0,23 | 0,23 | 0,34 | 404 |
| AI583064 | 0,03 | 0,01 | 0,24 | 0,28 | 0,42 | 405 |
| R94626 | 0,01 | -0,25 | -0,01 | 0,25 | 0,35 | 406 |
| AI216612 | 0,03 | -0,20 | 0,03 | 0,42 | 0,35 | 407 |
| NM_015318.1 | 0,01 | -0,12 | 0,11 | 0,32 | 0,25 | 408 |
| AA426397 | 0,02 | -0,37 | -0,14 | 0,33 | 0,34 | 409 |
| H78362 | 0,01 | -0,53 | -0,30 | 0,31 | 0,27 | 410 |
| AA878269 | 0,02 | -0,28 | -0,05 | 0,32 | 0,34 | 411 |
| NM_017778 | 0,01 | -0,17 | 0,06 | 0,39 | 0,26 | 412 |
| AI709236 | 0,00 | -0,24 | -0,01 | 0,21 | 0,28 | 413 |
| AA465175 | 0,01 | -0,11 | 0,12 | 0,32 | 0,27 | 414 |
| AI798573 | 0,00 | -0,34 | -0,12 | 0,18 | 0,21 | 415 |
| NM_139070 | 0,02 | -0,87 | -0,64 | 0,41 | 0,27 | 416 |
| XM_049749 | 0,04 | -0,18 | 0,05 | 0,50 | 0,28 | 417 |
| AI864931 | 0,01 | 0,09 | 0,31 | 0,24 | 0,31 | 418 |
| NM 021975 | 0,00 | -0,63 | -0,41 | 0,29 | 0,22 | 419 |
| R60931 | 0,03 | 0,16 | 0,39 | 0,35 | 0,35 | 420 |
| XM_037260 | 0,00 | -0,38 | -0,15 | 0,18 | 0,26 | 421 |
| R36650 | 0,00 | -0,40 | -0,17 | 0,23 | 0,25 | 422 |
| AA621075 | 0,00 | -0,31 | -0,09 | 0,24 | 0,28 | 423 |
| AI018273 | 0,04 | -0,23 | 0,00 | 0,45 | 0,31 | 424 |
| A1701905 | 0,00 | -0,24 | -0,01 | 0,22 | 0,24 | 425 |
| XM 054686 | 0,02 | -0,55 | -0,33 | 0,30 | 0,31 | 426 |
| NM_139276 | 0,02 | -0,09 | 0,14 | 0,25 | 0,38 | 427 |
| A1418064 | 0,01 | -0,23 | -0,01 | 0,32 | 0,24 | 428 |
| NM 002503 | 0,03 | -0,50 | -0,28 | 0,40 | 0,32 | 429 |
| AI923559 | 0.02 | -0,37 | -0,15 | 0,35 | 0,33 | 430 |
| NM_004295 | 0,04 | -0,66 | -0,44 | 0,40 | 0,37 | 431 |
| AA425105 | 0,03 | -0,19 | 0,04 | 0,43 | 0,29 | 432 |
| NM 002997 | 0,02 | 0,01 | 0,24 | 0,37 | 0,28 | 433 |
| NM_024013 | 0,00 | 0.04 | 0,27 | 0,28 | 0,23 | 434 |
| AA856755 | 0,02 | -0,40 | -0,18 | 0,34 | 0,34 | 435 |
| AI371339 | 0,00 | -0,28 | -0,06 | 0,24 | 0,22 | 436 |
| AA453528 | 0,04 | -0,30 | -0,08 | 0,50 | 0,27 | 437 |
| AI214646 | 0,04 | -0,27 | -0,05 | 0,24 | 0,43 | 438 |
| NM_006724 | 0,01 | -0.45 | -0,22 | 0,32 | 0,24 | 439 |
| AI925740 | 0,02 | 0,02 | 0,24 | 0,42 | 0,25 | 440 |
| H81378 | 0,00 | -0,16 | 0,06 | 0,32 | 0,19 | 441 |
| H82860 | 0,03 | 0,01 | 0,23 | 0,25 | 0,41 | 442 |
| BC032713 | 0,02 | 0,39 | 0,61 | 0,22 | 0,37 | 443 |
| H10036 | 0,05 | -0,08 | 0,13 | 0,42 | 0,35 | 444 |
| AI707917 | 0,00 | -0,34 | -0,12 | 0,18 | 0,20 | 445 |
| AA676928 | 0,05 | -0,04 | 0,18 | 0,36 | 0,40 | 446 |
| AI057616 | 0,00 | -0,03 | 0,19 | 0,19 | 0,30 | 447 |
| NM_003080 | 0,01 | -0.03 | 0,18 | 0,29 | 0,25 | 448 |
| AI685198 | 0,00 | -0,41 | -0,20 | 0,19 | 0,23 | 449 |
| AA436683 | 0,02 | -0,16 | 0,05 | 0,34 | 0,29 | 450 |
| R39456 | 0,00 | -0,34 | -0,13 | 0,17 | 0,23 | 451 |
| NM_004050 | 0,03 | -0,23 | -0,02 | 0,38 | 0,31 | 452 |
| N49208 | 0,02 | 0,13 | 0,34 | 0,35 | 0,32 | 453 |
| XM_055699 | 0,05 | 0,02 | 0,23 | 0,36 | 0,39 | 454 |
| BC028234 | 0,02 | -0,45 | -0,24 | 0,26 | 0,37 | 455 |
| N89900 | 0,02 | -0,39 | -0,18 | 0,36 | 0,28 | 456 |
| NM_001278 | 0,00 | -0.63 | -0,42 | 0,22 | 0,21 | 457 |
| AI921613 | 0,01 | -0,06 | 0,16 | 0,25 | 0,26 | 458 |
| NM_003821 | 0,03 | -0,64 | -0,43 | 0,43 | 0,23 | 459 |
| XM_046035 | 0,00 | -0,37 | -0,16 | 0,20 | 0,27 | 460 |
| AI936300 | 0,04 | 0,08 | 0,29 | 0,30 | 0,39 | 461 |
| NM_003131 | 0,00 | -0,71 | -0,50 | 0,30 | 0,21 | 462 |
| R61546 | 0,01 | -0,52 | -0,31 | 0,30 | 0,25 | 463 |
| AA431750 | 0,02 | -0,24 | -0,03 | 0,32 | 0,29 | 464 |
| AI524099 | 0,00 | -0,03 | 0,18 | 0.15 | 0,20 | 465 |
| XM_042665 | 0,00 | 0,07 | 0,28 | 0,25 | 0,22 | 466 |
| AI820873 | 0,02 | -0,48 | -0,27 | 0,39 | 0,24 | 467 |
| NM_019011 | 0,02 | -0,60 | -0,39 | 0,27 | 0,35 | 468 |
| H51585 | 0,05 | -0,57 | -0,36 | 0,42 | 0,30 | 469 |
| AI393173 | 0,02 | -0,04 | 0,16 | 0,25 | 0,34 | 470 |
| AI560205 | 0,00 | -0,36 | -0,15 | 0,19 | 0,23 | 471 |
| AA429020 | 0,00 | -0,31 | -0,11 | 0,20 | 0,17 | 472 |
| NM_000681.2 | 0,01 | 0,14 | 0,34 | 0,30 | 0,27 | 473 |
| NM_014550 | 0.00 | -0,10 | 0,10 | 0,25 | 0,16 | 474 |
| AA453256 | 0,00 | -0,04 | 0.16 | 0,20 | 0,26 | 475 |
| NM_021138 | 0,00 | -0,23 | -0,03 | 0,28 | 0,14 | 476 |
| R51304 | 0,05 | -0,02 | 0,19 | 0,32 | 0,38 | 477 |
| A1590111 | 0,00 | -0,31 | -0,10 | 0,14 | 0,20 | 478 |
| H09305 | 0,01 | -0,57 | -0,37 | 0,31 | 0,26 | 479 |
| R99076 | 0,00 | -0,40 | -0,19 | 0,19 | 0,22 | 480 |
| AI559096 | 0,01 | 0,28 | 0.48 | 0,29 | 0,29 | 481 |
| AI610213 | 0,02 | -0,12 | 0,08 | 0,34 | 0,27 | 482 |
| N66038 | 0,00 | -0,33 | -0,12 | 0,17 | 0,20 | 483 |
| NM_002649 | 0,00 | -0,33 | -0,13 | 0,17 | 0,29 | 484 |
| NM 006676 | 0,02 | -0,54 | -0,34 | 0,32 | 0,27 | 485 |
| NM 014959 | 0,00 | -0,38 | -0,18 | 0,24 | 0,25 | 486 |
| BC013992 | 0,01 | 0,01 | 0,21 | 0,16 | 0,32 | 487 |
| N32057 | 0.02 | -0,34 | -0,14 | 0.39 | 0,20 | 488 |
| AI801695 | 0,00 | -0,33 | -0,13 | 0,17 | 0,18 | 489 |
| AI568793 | 0,03 | 0,11 | 0,31 | 0,29 | 0,33 | 490 |
| AA479285 | 0,00 | 0,08 | 0,27 | 0,23 | 0,23 | 491 |
| H06501 | 0,02 | -0,10 | 0,10 | 0,32 | 0.28 | 492 |
| R00259 | 0,03 | -0,06 | 0,14 | 0.31 | 0,32 | 493 |
| AI362368 | 0,00 | -0,33 | -0,13 | 0,17 | 0,19 | 494 |
| AI635040 | 0,00 | -0,14 | 0,06 | 0,18 | 0,26 | 495 |
| AI354869 | 0,03 | -0,48 | -0,29 | 0,32 | 0,26 | 496 |
| N71407 | 0,02 | 0,06 | 0,25 | 0,26 | 0,32 | 497 |
| XM 038544 | 0,04 | -0,12 | 0,07 | 0,38 | 0,29 | 498 |
| NM 031910 | 0,04 | 0,18 | 0,38 | 0,27 | 0,38 | 499 |
| AI862063 | 0,00 | -0,28 | -0,08 | 0,20 | 0,24 | 500 |
| AA455638 | 0,03 | 0,07 | 0,27 | 0,28 | 0,32 | 501 |
| AI697430 | 0,00 | -0,36 | -0,17 | 0,18 | 0,21 | 502 |
| R42480 | 0,01 | -0,49 | -0,29 | 0,25 | 0,23 | 503 |
| AI674115 | 0,01 | 0,02 | 0,21 | 0,24 | 0,29 | 504 |
| AA968926 | 0,03 | -0,32 | -0,13 | 0,37 | 0,26 | 505 |
| AI524694 | 0,00 | -0,38 | -0,19 | 0,18 | 0,23 | 506 |
| AA609857 | 0,02 | -0.08 | 0,12 | 0,30 | 0,29 | 507 |
| AI913713 | 0,01 | -0,46 | -0,27 | 0,33 | 0,21 | 508 |
| W04695 | 0,00 | -0,28 | -0,09 | 0,16 | 0,24 | 509 |
| NM 033012 | 0,04 | -0,12 | 0,07 | 0,35 | 0,31 | 510 |
| T77048 | 0,02 | -0,01 | 0,18 | 0,26 | 0,31 | 511 |
| AI817381 | 0,01 | -0,25 | -0,06 | 0,23 | 0,24 | 512 |
| AI624918 | 0,03 | -0,02 | 0,17 | 0,28 | 0,32 | 513 |
| AI888072 | 0,01 | -0,26 | -0,07 | 0,23 | 0,26 | 514 |
| AA883759 | 0,00 | -0,38 | -0,20 | 0,21 | 0,22 | 515 |
| AA478611 | 0,00 | -0,34 | -0,15 | 0,25 | 0,17 | 516 |
| AI452862 | 0,03 | -0,28 | -0.09 | 0,34 | 0,26 | 517 |
| AI277955 | 0,00 | -0,46 | -0,27 | 0,24 | 0,22 | 518 |
| AI520967 | 0,00 | -0,34 | -0,15 | 0,17 | 0,20 | 519 |
| T91937 | 0,05 | 0.36 | 0,54 | 0,27 | 0,37 | 520 |
| AA993698 | 0,00 | 0,01 | 0,20 | 0,21 | 0,20 | 521 |
| AI620374 | 0,00 | -0,40 | -0,22 | 0,18 | 0,22 | 522 |
| AA707628 | 0,00 | -0,27 | -0,08 | 0,12 | 0,17 | 523 |
| AI572545 | 0,01 | -0,38 | -0,19 | 0,17 | 0,28 | 524 |
| AI801540 | 0,04 | -0,16 | 0,03 | 0,36 | 0,28 | 525 |
| A1354889 | 0,00 | -0,11 | 0,07 | 0,22 | 0,18 | 526 |
| NM_030751 | 0,03 | -0,09 | 0,09 | 0,33 | 0,25 | 527 |
| NM_000657 | 0,01 | -0,50 | -0,32 | 0,27 | 0,22 | 528 |
| AA045139 | 0,02 | -0,43 | -0,24 | 0,34 | 0,23 | 529 |
| AI912148 | 0,00 | -0,25 | -0,06 | 0,18 | 0,20 | 530 |
| AA513806 | 0,04 | -0,21 | -0,03 | 0,29 | 0,32 | 531 |
| H48440 | 0,00 | -0,35 | -0,17 | 0,16 | 0,23 | 532 |
| AA114117 | 0,00 | -0,38 | -0,20 | 0,17 | 0,18 | 533 |
| A1654471 | 0,00 | -0,20 | ₋0,02 | 0,19 | 0,20 | 534 |
| AA423792 | 0,00 | -0,16 | 0,02 | 0,14 | 0,25 | 535 |
| AI926484 | 0,00 | -0,08 | 0,10 | 0,25 | 0,14 | 536 |
| T89979 | 0,00 | -0,30 | -0,12 | 0,16 | 0,19 | 537 |
| AI889310 | 0,01 | -0,25 | -0,07 | 0,26 | 0,21 | 538 |
| R11261 | 0,04 | -0,27 | -0,09 | 0,43 | 0,18 | 539 |
| AI932551 | 0.00 | -0,32 | -0,14 | 0,16 | 0,23 | 540 |
| NM_017626.1 | 0,01 | -0,56 | -0,38 | 0,22 | 0,26 | 541 |
| AI381513 | 0,04 | -0,29 | -0,11 | 0,32 | 0,29 | 542 |
| AA682407 | 0,02 | -0,24 | -0,07 | 0,25 | 0,29 | 543 |
| AA954316 | 0,04 | -0,75 | -0,57 | 0,35 | 0,27 | 544 |
| AI791500 | 0,02 | 0,16 | 0,34 | 0,18 | 0,31 | 545 |
| T91881 | 0,00 | -0,26 | -0,08 | 0,18 | 0,23 | 546 |
| AI149857 | 0,02 | -0,06 | 0,12 | 0,18 | 0.30 | 547 |
| AI370842 | 0,04 | 0,11 | 0,29 | 0,31 | 0,30 | 548 |
| AA401205 | 0,00 | -0,13 | 0,05 | 0,18 | 0,19 | 549 |
| AA453267 | 0,02 | -0,03 | 0,15 | 0,26 | 0,28 | 550 |
| R88475 | 0,00 | -0,35 | -0,17 | 0,18 | 0,20 | 551 |
| Ai864919 | 0,01 | -0,38 | -0,20 | 0,19 | 0,25 | 552 |
| NM_002169 | 0,04 | -0,24 | -0,07 | 0,33 | 0,27 | 553 |
| R46801 | 0,05 | 0,27 | 0,44 | 0,35 | 0,27 | 554 |
| AI277856 | 0,02 | -0,12 | 0,06 | 0,22 | 0,27 | 555 |
| H22921 | 0,00 | -0,33 | -0,15 | 0,19 | 0,22 | 556 |
| AI763386 | 0,03 | -0,37 | -0,20 | 0,30 | 0,27 | 557 |
| N78812 | 0,01 | -0,23 | -0,06 | 0,25 | 0,20 | 558 |
| H83981 | 0,04 | 0,04 | 0,22 | 0,28 | 0,30 | 559 |
| AA029887 | 0,00 | -0,40 | -0,22 | 0,19 | 0,21 | 560 |
| AI192112 | 0,00 | -0,11 | 0,06 | 0,15 | 0,24 | 561 |
| W88960 | 0,01 | 0,10 | 0,28 | 0,21 | 0,24 | 562 |
| W80744 | 0,00 | -0,25 | -0,08 | 0,15 | 0,21 | 563 |
| AI521577 | 0,01 | -0,31 | -0,13 | 0,18 | 0,23 | 564 |
| AA418572 | 0,01 | -0,13 | 0,05 | 0,17 | 0,25 | 565 |
| N73510 | 0,00 | -0,38 | -0,21 | 0,17 | 0,22 | 566 |
| AI631299 | 0,03 | -0,16 | 0,01 | 0,24 | 0,29 | 567 |
| XM_012717 | 0,00 | -0,44 | -0,27 | 0,18 | 0,17 | 568 |
| NM_000590 | 0,03 | 0,32 | 0,50 | 0,23 | 0,29 | 569 |
| AI381910 | 0,01 | -0,04 | 0,13 | 0,21 | 0,23 | 570 |
| R87714 | 0,04 | -0,18 | -0,01 | 0,33 | 0,23 | 571 |
| AA609628 | 0,00 | -0,36 | -0,19 | 0,17 | 0,19 | 572 |
| AA634317 | 0,03 | 0.19 | 0,36 | 0,27 | 0,28 | 573 |
| AI214830 | 0,04 | -0,27 | -0,10 | 0,23 | 0,32 | 574 |
| AI203201 | 0,04 | -0,26 | -0,09 | 0,26 | 0,29 | 575 |
| AI924806 | 0,00 | -0,29 | -0,12 | 0,20 | 0,18 | 576 |
| AA701319 | 0,02 | -0,07 | 0,10 | 0,22 | 0,28 | 577 |
| N63628 | 0,03 | -0,26 | -0,09 | 0,26 | 0,27 | 578 |
| R02742 | 0,04 | -0,17 | -0,01 | 0,32 | 0,25 | 579 |
| H07860 | 0,02 | -0,03 | 0,13 | 0,26 | 0,24 | 580 |
| H77534 | 0,02 | -0,35 | -0,18 | 0,32 | 0,20 | 581 |
| AI208537 | 0,02 | -0,21 | -0,04 | 0,34 | 0,17 | 582 |
| AI184715 | 0,01 | -0,03 | 0,13 | 0,23 | 0,20 | 583 |
| R05816 | 0,00 | -0,27 | -0,10 | 0,19 | 0,20 | 584 |
| AA961252 | 0,04 | -0,14 | 0,02 | 0,26 | 0,31 | 585 |
| AI801425 | 0,00 | -0,21 | -0,04 | 0,22 | 0,17 | 586 |
| AA477776 | 0,01 | -0,01 | 0,16 | 0,21 | 0,20 | 587 |
| R06585 | 0,01 | -0,40 | -0,23 | 0,18 | 0,21 | 588 |
| AA405788 | 0,01 | -0,36 | -0,19 | 0,15 | 0,25 | 589 |
| R06107 | 0,01 | -0,23 | -0,07 | 0,24 | 0,19 | 590 |
| AA923316 | 0,00 | -0,20 | -0,04 | 0,15 | 0,19 | 591 |
| AI421397 | 0,02 | -0,02 | 0,14 | 0,19 | 0,26 | 592 |
| NM 006881 | 0,01 | -0,40 | -0,24 | 0,20 | 0,23 | 593 |
| R43415 | 0,00 | -0,24 | -0,08 | 0,14 | 0,19 | 594 |
| H11495 | 0,01 | -0,29 | -0,12 | 0,26 | 0,13 | 595 |
| AI208772 | 0,04 | -0,23 | -0.07 | 0,29 | 0,27 | 596 |
| AA479784 | 0,03 | -0,06 | 0,10 | 0,29 | 0,24 | 597 |
| AA485092 | 0,00 | -0,36 | -0,20 | 0,16 | 0,20 | 598 |
| AA664688 | 0,00 | -0,39 | -0,23 | 0,18 | 0,20 | 599 |
| H48230 | 0,01 | -0,29 | -0,13 | 0,19 | 0,21 | 600 |
| AI248075 | 0,02 | -0,12 | 0,04 | 0,25 | 0,22 | 601 |
| AA418695 | 0,04 | -0,01 | 0,15 | 0,21 | 0,31 | 602 |
| AI673731 | 0,01 | -0,41 | -0,25 | 0,16 | 0,22 | 603 |
| XM_008948 | 0,03 | 0,10 | 0,26 | 0,26 | 0,26 | 604 |
| AI301257 | 0,00 | -0,31 | -0,15 | 0,19 | 0,19 | 605 |
| NM 003823 | 0,04 | -0,72 | -0,56 | 0,31 | 0,24 | 606 |
| AI744264 | 0,01 | -0,14 | 0,02 | 0,16 | 0,22 | 607 |
| AI809873 | 0,03 | -0,45 | -0,29 | 0,24 | 0,26 | 608 |
| AI354243 | 0,01 | -0,34 | -0,18 | 0,17 | 0,21 | 609 |
| NM 001553.1 | 0,04 | -0,15 | 0,01 | 0,27 | 0,27 | 610 |
| W86575 | 0,02 | -0,34 | -0,18 | 0,23 | 0,24 | 611 |
| AA442720 | 0,03 | -0,15 | 0,01 | 0,27 | 0,24 | 612 |
| AA993597 | 0.03 | 0,17 | 0,33 | 0,26 | 0,24 | 613 |
| AI433952 | 0,01 | -0,30 | -0,14 | 0,16 | 0,23 | 614 |
| R56800 | 0,01 | -0,09 | 0,06 | 0,17 | 0,21 | 615 |
| AA417031 | 0,01 | -0,21 | -0,06 | 0,19 | 0,23 | 616 |
| R53961 | 0,04 | -0,45 | -0,29 | 0,28 | 0,25 | 617 |
| T86887 | 0,00 | -0,23 | -0,08 | 0,13 | 0,20 | 618 |
| AA705808 | 0,01 | -0,20 | -0,04 | 0,25 | 0,18 | 619 |
| AA426451 | 0,00 | -0,28 | -0,13 | 0.16 | 0,19 | 620 |
| H06263 | 0,00 | -0,28 | -0,12 | 0,14 | 0.17 | 621 |
| AA659421 | 0,00 | -0,32 | -0,17 | 0,14 | 0,16 | 622 |
| AI801595 | 0,00 | -0,28 | -0,13 | 0,16 | 0,19 | 623 |
| AI672318 | 0.04 | -0,20 | -0.05 | 0.31 | 0,24 | 624 |
| AI762019 | 0.01 | -0,25 | -0,09 | 0,19 | 0,21 | 625 |
| N92873 | 0,02 | -0,11 | 0,05 | 0,28 | 0,19 | 626 |
| NM 017442 | 0,04 | 0,08 | 0,23 | 0,28 | 0,25 | 627 |
| H46164 | 0,03 | 0,03 | 0,18 | 0,21 | 0,27 | 628 |
| T83946 | 0,01 | -0.29 | -0.14 | 0,20 | 0,21 | 629 |
| AA868726 | 0,04 | -0,42 | -0,27 | 0,26 | 0,25 | 630 |
| H88129 | 0,02 | -0,37 | -0,22 | 0,21 | 0,23 | 631 |
| R88267 | 0,04 | -0,12 | 0,03 | 0,30 | 0,23 | 632 |
| AI798545 | 0,01 | -0,32 | -0,17 | 0,17 | 0,19 | 633 |
| N57775 | 0,02 | -0,14 | 0,01 | 0,22 | 0,22 | 634 |
| AA425134 | 0,00 | -0,21 | -0,07 | 0,16 | 0,19 | 635 |
| AI744807 | 0,01 | -0,59 | -0,44 | 0,20 | 0,22 | 636 |
| AI702056 | 0,05 | -0,27 | -0,12 | 0,22 | 0,29 | 637 |
| NM_000575 | 0,04 | -0,27 | -0,12 | 0,23 | 0,25 | 638 |
| T98779 | 0,01 | -0,38 | -0,23 | 0,18 | 0,23 | 639 |
| NM_000587 | 0,01 | -0,43 | -0,28 | 0,18 | 0,19 | 640 |
| R92455 | 0,01 | -0,36 | -0,21 | 0,17 | 0,21 | 641 |
| AI758473 | 0,01 | -0,36 | -0,21 | 0,18 | 0,22 | 642 |
| AA398364 | 0,00 | -0,31 | -0,17 | 0,13 | 0,21 | 643 |
| AI811774 | 0,05 | 0,20 | 0,35 | 0,23 | 0,27 | 644 |
| AI299411 | 0,00 | -0,24 | -0,10 | 0,17 | 0,18 | 645 |
| AA225138 | 0,00 | -0,26 | -0,11 | 0,12 | 0,17 | 646 |
| AA418689 | 0,05 | 0,09 | 0,24 | 0,21 | 0,28 | 647 |
| T77995 | 0,01 | -0,18 | -0,04 | 0,21 | 0,20 | 648 |
| AA808788 | 0,04 | -0,33 | -0,18 | 0,18 | 0,25 | 649 |
| AI677645 | 0,01 | -0,25 | -0,11 | 0,15 | 0,19 | 650 |
| AA629306 | 0,04 | -0,07 | 0,07 | 0,26 | 0,24 | 651 |
| AA749151 | 0,00 | -0,19 | -0,05 | 0,13 | 0,17 | 652 |
| AI679294 | 0,01 | -0,41 | -0,27 | 0,19 | 0,19 | 653 |
| R45611 | 0,02 | -0,24 | -0,10 | 0,16 | 0,24 | 654 |
| NM_000588 | 0,05 | -0,18 | -0,04 | 0,29 | 0,22 | 655 |
| H99483 | 0,01 | -0,29 | -0,15 | 0,16 | 0,22 | 656 |
| AI679923 | 0,01 | -0,46 | -0,32 | 0,17 | 0,20 | 657 |
| AI077580 | 0,05 | -0,04 | 0,10 | 0,27 | 0,23 | 658 |
| D49410 | 0,01 | -0,31 | -0,17 | 0,19 | 0,19 | 659 |
| AI692267 | 0,04 | -0,42 | -0,28 | 0,22 | 0,24 | 660 |
| AI804001 | 0,02 | 0,00 | 0,14 | 0,19 | 0,23 | 661 |
| T87188 | 0,01 | -0,32 | -0,18 | 0,19 | 0,19 | 662 |
| AI368218 | 0,02 | -0,24 | -0,10 | 0,13 | 0,23 | 663 |
| AI208749 | 0,02 | -0,02 | 0,11 | 0,22 | 0,19 | 664 |
| H61046 | 0,02 | -0,21 | -0.07 | 0,18 | 0,22 | 665 |
| NM_001330.1 | 0,01 | -0,05 | 0,08 | 0,19 | 0,20 | 666 |
| XM_001322 | 0,01 | -0,33 | -0,19 | 0,18 | 0,17 | 667 |
| NM_M4195 | 0,04 | 0,23 | 0,37 | 0,16 | 0.27 | 668 |
| AI285713 | 0,01 | -0,32 | -0,18 | 0,15 | 0,21 | 669 |
| AA527369 | 0,00 | -0,13 | 0,00 | 0,15 | 0,16 | 670 |
| AI350069 | 0,01 | -0,24 | -0,11 | 0,15 | 0,21 | 671 |
| AI493975 | 0,01 | -0,24 | -0,10 | 0,18 | 0,16 | 672 |
| AI355007 | 0,03 | -0.23 | -0.10 | 0,22 | 0,21 | 673 |
| AA225239 | 0,04 | -0,40 | -0,26 | 0,21 | 0,25 | 674 |
| AA001392 | 0,03 | -0,39 | -0,26 | 0,24 | 0,19 | 675 |
| AI933797 | 0,02 | -0,28 | -0,15 | 0,22 | 0,18 | 676 |
| R43065 | 0,01 | -0,21 | -0,08 | 0,16 | 0,18 | 677 |
| AA478621 | 0,03 | -0,21 | -0,08 | 0,21 | 0,20 | 678 |
| AA012850 | 0,03 | -0,32 | -0,19 | 0,17 | 0,22 | 679 |
| AI925035 | 0,03 | -0,15 | -0,02 | 0,17 | 0,23 | 680 |
| AA995218 | 0,03 | -0,22 | -0,09 | 0,19 | 0,21 | 681 |
| AA897716 | 0,04 | -0,18 | -0,06 | 0,23 | 0,21 | 682 |
| AA983987 | 0,02 | -0,28 | -0,15 | 0,18 | 0,18 | 683 |
| AI762202 | 0,03 | -0,18 | -0,05 | 0,22 | 0,20 | 684 |
| T95909 | 0,02 | -0,34 | -0.22 | 0,18 | 0,19 | 685 |
| N22551 | 0,03 | -0,36 | -0,24 | 0,17 | 0,22 | 686 |
| AI769053 | 0.03 | -0,28 | -0,15 | 0,15 | 0,22 | 687 |
| AF039955 | 0,01 | -0,37 | -0,24 | 0,20 | 0,16 | 688 |
| AI935874 | 0,02 | -0,26 | -0,14 | 0,16 | 0,20 | 689 |
| AI570779 | 0,01 | -0,31 | -0,19 | 0,15 | 0,18 | 690 |
| AI240539 | 0,01 | -0.22 | -0,09 | 0,17 | 0,18 | 691 |
| H54423 | 0,03 | -0,32 | -0,20 | 0,16 | 0.22 | 692 |
| AA460136 | 0,02 | -0,09 | 0,03 | 0,24 | 0,14 | 693 |
| NM_033357 | 0,05 | -0,24 | -0,12 | 0,19 | 0,23 | 694 |
| AI923479 | 0,04 | -0,30 | -0,18 | 0,20 | 0,22 | 695 |
| H18944 | 0,04 | -0,42 | -0,30 | 0,21 | 0,19 | 696 |
| NM 006509 | 0,03 | 0,01 | 0,13 | 0,12 | 0,23 | 697 |
| AI865298 | 0,02 | -0,31 | -0,19 | 0,13 | 0,20 | 698 |
| AI123502 | 0,04 | -0,36 | -0,24 | 0,17 | 0,22 | 699 |
| AI885918 | 0,02 | -0,24 | -0,12 | 0,14 | 0,19 | 700 |
| AA225023 | 0,02 | -0,33 | -0,21 | 0,12 | 0,20 | 701 |
| AA421020 | 0,04 | -0,25 | -0,13 | 0,19 | 0,21 | 702 |
| AJ297560 | 0,05 | -0,29 | -0,17 | 0,21 | 0,20 | 703 |
| N95217 | 0,02 | -0,30 | -0,19 | 0,12 | 0.19 | 704 |
| AA526032 | 0,04 | -0,25 | -0,13 | 0,20 | 0,19 | 705 |
| AA496309 | 0,02 | -0.32 | -0,20 | 0,15 | 0,19 | 706 |
| AI732958 | 0,03 | -0,22 | -0.11 | 0,18 | 0.182 | 707 |
| AA410828 | 0,02 | -0,29 | -0,18 | 0,20 | 0,16 | 708 |
| AA453993 | 0,02 | -0,30 | -0,19 | 0,18 | 0,16 | 709 |
| R92993 | 0,02 | -0,26 | -0,15 | 0,12 | 0,19 | 710 |
| NM_003921 | 0,04 | -0,23 | -0,13 | 0,20 | 0,18 | 711 |
| AI379967 | 0,02 | -0.34 | -0,23 | 0,15 | 0,17 | 712 |
| AI926656 | 0,04 | -0,26 | -0,15 | 0,18 | 0,19 | 713 |
| AA935872 | 0,03 | -0,31 | -0,20 | 0.16 | 0,18 | 714 |
| H08791 | 0,03 | -0,27 | -0,17 | 0,16 | 0,18 | 715 |
| AI932884 | 0,03 | -0,31 | -0,21 | 0,16 | 0,18 | 716 |
| AI926745 | 0,03 | -0,33 | -0,22 | 0,18 | 0,16 | 717 |
| R99595 | 0,05 | -0,29 | -0.19 | 0,16 | 0,20 | 718 |
| AI824579 | 0,03 | -0.31 | -0,21 | 0,13 | 0,18 | 719 |
| AA427886 | 0,03 | -0,27 | -0,17 | 0,14 | 0,16 | 720 |
| H42488 | 0.04 | -0.33 | -0,24 | 0,16 | 0,15 | 721 |

**Tabelle 3: Signifikant reduzierte Genaktivitäten in Proben von Patienten mit infektiösem MODS/MOV, im Vergleich zu den Genaktivitäten von Patienten mit nichtinfektiösem MODS/MOV**

| **GenBank Accession-Number** | **p-Wert** | **Mittlerer normalisierter und transformierter Expressionswert** | | **Standardabweichung** | | **Sequenz-ID** |
|---|---|---|---|---|---|---|
| | | **Gruppe der Patienten mit nichtinfektiösem /MOV** | **Gruppe der Patienten mit infektiösem MOV** | **Gruppe der Patienten nichtinfektiösem /MOV** | **Gruppe der Patienten mit infektiösem MOV** | |
| NM_019111 | 0.00 | 1,41 | 0,21 | 0,73 | 0,56 | 722 |
| N29761 | 0,00 | -0,25 | -1,35 | 0,61 | 0,92 | 723 |
| NM_002124 | 0,00 | 1,60 | 0,54 | 0,62 | 0.52 | 724 |
| R43910 | 0,00 | 2,51 | 1,49 | 1,25 | 0,88 | 725 |
| NM_000570 | 0,00 | 3,66 | 2,67 | 0,70 | 1,31 | 726 |
| NM_002923 | 0,00 | 2,03 | 1,07 | 0,83 | 0,89 | 727 |
| X00457 | 0,00 | 1,46 | 0,50 | 0,84 | 0,60 | 728 |
| NM_022555 | 0,00 | 1,86 | 0,91 | 0,58 | 0,54 | 729 |
| NM_002125 | 0,00 | 1,38 | 0,46 | 0,55 | 0,45 | 730 |
| AA620760 | 0,00 | 0,30 | -0,62 | 0,47 | 0,60 | 731 |
| NM_000569 | 0,01 | 3,13 | 2,26 | 0,86 | 1,21 | 732 |
| NM_021983 | 0,00 | 1,38 | 0,52 | 0,48 | 0,41 | 733 |
| R43203 | 0,00 | 2,03 | 1,18 | 1,16 | 0,74 | 734 |
| NM_033554 | 0,00 | 1,42 | 0,60 | 0,60 | 0,52 | 735 |
| AA626239 | 0,00 | 0,15 | -0,65 | 0,74 | 0,73 | 736 |
| NM_007328 | 0,00 | -0,31 | -1,10 | 0,49 | 0,64 | 737 |
| M90746 | 0,02 | 3,67 | 2,89 | 0,74 | 1,37 | 738 |
| T91086 | 0,00 | -0,81 | -1,59 | 0,63 | 0,67 | 739 |
| AA151104 | 0,00 | 0,11 | -0,64 | 0,46 | 0,46 | 740 |
| H45298 | 0,01 | 1,84 | 1,09 | 0,90 | 0,97 | 741 |
| NM_031311 | 0,00 | 0,78 | 0,03 | 0,52 | 0,39 | 742 |
| AI590144 | 0,00 | 1,70 | 0,96 | 0,97 | 0,70 | 743 |
| NM_001824.2 | 0,00 | 1,63 | 0,88 | 0,58 | 0,67 | 744 |
| NM_018643 | 0,00 | 1,70 | 0,96 | 0,54 | 0,66 | 745 |
| AA400790 | 0,00 | 0,93 | 0,20 | 0,59 | 0,52 | 746 |
| NM_001251 | 0,00 | 1.18 | 0,47 | 0,42 | 0,48 | 747 |
| NM_000887.2 | 0,00 | 0,24 | -0,48 | 0,92 | 0,61 | 748 |
| AI696291 | 0,00 | 0,74 | 0,04 | 0,64 | 0.39 | 749 |
| NM_031477 | 0.02 | 1.89 | 1.19 | 1,02 | 1,06 | 750 |
| AA910846 | 0,00 | 1,29 | 0,60 | 0,87 | 0,45 | 751 |
| NM_005538 | 0.00 | 1,59 | 0,91 | 0,90 | 0,72 | 752 |
| AA398331 | 0,00 | 0,13 | -0,53 | 0,51 | 0,46 | 753 |
| NM_025139 | 0,04 | -1,79 | -2,41 | 0,75 | 1,17 | 754 |
| AA398611 | 0,00 | 1.16 | 0,54 | 0,77 | 0,43 | 755 |
| NM_006682 | 0,00 | -0,05 | -0,67 | 0,46 | 0,39 | 756 |
| X52473 | 0,00 | 1,71 | 1,09 | 0,59 | 0,74 | 757 |
| AI859777 | 0,01 | -1,02 | -1,63 | 0,87 | 0,77 | 758 |
| HI8649 | 0,00 | -0.41 | -1,01 | 0,30 | 0,58 | 759 |
| AI700444 | 0,00 | 1,57 | 0,97 | 0,70 | 0,63 | 760 |
| XM_001472 | 0,00 | -0,36 | -0,95 | 0,61 | 0,59 | 761 |
| XM_049959 | 0,01 | 2,10 | 1.53 | 0,73 | 0,78 | 762 |
| AA863064 | 0,03 | 0,96 | 0,39 | 1,20 | 0,63 | 763 |
| H88328 | 0,01 | -1.27 | -1,84 | 0,73 | 0,69 | 764 |
| R40861 | 0,00 | 0,82 | 0,25 | 0,82 | 0,51 | 765 |
| AI733498 | 0,00 | -0.37 | -0.93 | 0,36 | 0,58 | 766 |
| NM_002621 | 0,01 | 1,16 | 0,61 | 0,63 | 0,70 | 767 |
| AI732971 | 0,00 | 0,46 | -0,09 | 0,55 | 0,35 | 768 |
| AA813145 | 0,00 | 0,48 | -0,07 | 0,40 | 0,41 | 769 |
| NM_004221.2 | 0,02 | 2,05 | 1,51 | 0,75 | 0,79 | 770 |
| AA740907 | 0,00 | 0,05 | -0,49 | 0,44 | 0,32 | 771 |
| NM_032022 | 0,00 | 0,90 | 0,36 | 0,42 | 0,50 | 772 |
| XM_003789 | 0,00 | -0.25 | -0,78 | 0,37 | 0,47 | 773 |
| AI357099 | 0,02 | -1,06 | -1,59 | 0,85 | 0,78 | 774 |
| NM_003937 | 0,00 | -0,43 | -0,95 | 0,44 | 0,44 | 775 |
| NM_002122 | 0,00 | 0,84 | 0,33 | 0,71 | 0,51 | 776 |
| AI625626 | 0,01 | 0,90 | 0,40 | 0,87 | 0,52 | 777 |
| H23819 | 0,00 | 0,97 | 0,46 | 0,62 | 0,47 | 778 |
| AI797009 | 0,01 | 0,64 | 0,14 | 0,85 | 0,57 | 779 |
| XM_031354 | 0,01 | 0,99 | 0,49 | 0,63 | 0,72 | 780 |
| XM_051958 | 0,01 | 1,20 | 0,70 | 0,53 | 0,72 | 781 |
| AI499173 | 0,01 | 0,11 | -0,38 | 0,75 | 0,50 | 782 |
| NM_000591 | 0,00 | 0,92 | 0,43 | 0,50 | 0,55 | 783 |
| NM_057158 | 0,00 | -0,03 | -0,52 | 0,49 | 0,34 | 784 |
| R71775 | 0,00 | 0,42 | -0,07 | 0,61 | 0,55 | 785 |
| AI924028 | 0,00 | -0,35 | -0,84 | 0,36 | 0,58 | 786 |
| R39504 | 0,00 | -0,50 | -0,98 | 0,34 | 0,40 | 787 |
| N66205 | 0,01 | 1,49 | 1.02 | 0,72 | 0,62 | 788 |
| AI738831 | 0,00 | 0,09 | -0,38 | 0,29 | 0,56 | 789 |
| H18435 | 0,00 | 0,34 | -0,14 | 0,43 | 0,32 | 790 |
| R39782 | 0,00 | -0,35 | -0,82 | 0.44 | 0,35 | 791 |
| R38717 | 0,00 | -0,16 | -0,63 | 0,43 | 0,48 | 792 |
| H96798 | 0,00 | 0,19 | -0,27 | 0,47 | 0,53 | 793 |
| N72174 | 0,02 | 0,92 | 0,46 | 0,53 | 0,73 | 794 |
| AI739381 | 0,00 | -0,11 | -0,57 | 0,24 | 0,50 | 795 |
| AI654546 | 0,00 | 0,05 | -0,41 | 0,31 | 0,46 | 796 |
| AI097494 | 0,00 | -0,72 | -1,19 | 0,41 | 0,57 | 797 |
| NM_000612.2 | 0,00 | 0,93 | 0,47 | 0,50 | 0,36 | 798 |
| AI651536 | 0,00 | 0,62 | 0,16 | 0,50 | 0,29 | 799 |
| AI804425 | 0,00 | 0,90 | 0,44 | 0,73 | 0,38 | 800 |
| n67686 | 0,00 | 0,43 | -0,03 | 0,62 | 0,38 | 801 |
| NM_000062 | 0,01 | -0,18 | -0,63 | 0,75 | 0,45 | 802 |
| R54442 | 0,00 | 0,69 | 0,24 | 0,53 | 0,39 | 803 |
| AI475085 | 0,00 | 0,25 | -0.20 | 0,67 | 0,29 | 804 |
| AI700612 | 0,01 | -0,85 | -1,30 | 0,60 | 0,61 | 805 |
| AA447615 | 0,00 | 0,18 | -0.27 | 0,60 | 0,30 | 806 |
| AI223092 | 0,00 | -0,38 | -0,82 | 0,30 | 0,56 | 807 |
| AI262894 | 0,00 | 0,47 | 0,03 | 0,61 | 0,39 | 808 |
| R52949 | 0,01 | -1,08 | -1,52 | 0,44 | 0,72 | 809 |
| AA629034 | 0,00 | -0,13 | -0,57 | 0,37 | 0,33 | 810 |
| R12559 | 0,00 | 0,72 | 0,29 | 0,45 | 0,37 | 811 |
| AA910310 | 0,00 | 0,03 | -0,40 | 0,35 | 0,27 | 812 |
| NM_006850 | 0,01 | 0,19 | -0,24 | 0,59 | 0,51 | 813 |
| AI689080 | 0,05 | 1,34 | 0,91 | 0,74 | 0,78 | 814 |
| R23755 | 0,00 | 0,16 | -0,26 | 0,40 | 0,37 | 815 |
| N95041 | 0,00 | 0,17 | -0,25 | 0,30 | 0,40 | 816 |
| AA443712 | 0,01 | 0,76 | 0,34 | 0,72 | 0,41 | 817 |
| NM_033302 | 0,03 | 1,53 | 1,11 | 0,54 | 0,72 | 818 |
| AI700810 | 0,00 | 0,59 | 0,18 | 0,72 | 0,25 | 819 |
| XM_004011 | 0,00 | 0,52 | 0,11 | 0,44 | 0,35 | 820 |
| H11433 | 0,00 | 0,38 | -0,03 | 0,55 | 0,35 | 821 |
| NM_006890 | 0,03 | 1,14 | 0,73 | 0,77 | 0,52 | 822 |
| NM_138556 | 0,00 | 0,16 | -0.25 | 0,23 | 0,36 | 823 |
| XM_003937 | 0,00 | 0,13 | -0,28 | 0,34 | 0,33 | 824 |
| NM_000908.1 | 0,00 | -0,05 | -0,46 | 0,22 | 0,30 | 825 |
| NM_017567 | 0,01 | -0,52 | -0,92 | 0,57 | 0,47 | 826 |
| R89802 | 0,00 | -0,21 | -0,61 | 0,27 | 0,33 | 827 |
| NM_000715 | 0,01 | 0,77 | 0,37 | 0.56 | 0,46 | 828 |
| AI924733 | 0,00 | -0,60 | -1,00 | 0,37 | 0,50 | 829 |
| AI859370 | 0,00 | 0,17 | -0,23 | 0,16 | 0,24 | 830 |
| AI023558 | 0,00 | -0,41 | -0,80 | 0,23 | 0,37 | 831 |
| AA021303 | 0,00 | 0,19 | -0,20 | 0,58 | 0,25 | 832 |
| R69609 | 0,01 | 1,03 | 0,64 | 0,54 | 0.51 | 833 |
| XM_057445 | 0,00 | 0,27 | -0,12 | 0,35 | 0,39 | 834 |
| AA046302 | 0,00 | -0,10 | -0,49 | 0,36 | 0,33 | 835 |
| AI383451 | 0,01 | 0,26 | -0,13 | 0,53 | 0,40 | 836 |
| AA464191 | 0,00 | -0,46 | -0,84 | 0,32 | 0,41 | 837 |
| AA425808 | 0,00 | -0,22 | -0,61 | 0,25 | 0,51 | 838 |
| XM_038024 | 0,00 | 0,18 | -0,21 | 0,28 | 0,47 | 839 |
| AI016127 | 0,01 | 1,07 | 0,69 | 0,56 | 0,42 | 840 |
| AA400144 | 0,03 | -0,41 | -0,79 | 0,60 | 0,62 | 841 |
| R43074 | 0,00 | -0,99 | -1,36 | 0,28 | 0,51 | 842 |
| AI628936 | 0,01 | -0,65 | -1,03 | 0,41 | 0,51 | 843 |
| AA461499 | 0,00 | -0,16 | -0,54 | 0,39 | 0,38 | 844 |
| AI668673 | 0,00 | 0,34 | -0,03 | 0,35 | 0,50 | 845 |
| AI539443 | 0,00 | 0,24 | -0,13 | 0,39 | 0,43 | 846 |
| AA404231 | 0,04 | -0,14 | -0,52 | 0,52 | 0,69 | 847 |
| AI692869 | 0,01 | 0,72 | 0,34 | 0,30 | 0,53 | 848 |
| AI822099 | 0,00 | 0,00 | -0,37 | 0,51 | 0,34 | 849 |
| R20616 | 0,00 | 0,12 | -0,25 | 0,30 | 0,32 | 850 |
| AA453406 | 0,01 | -0,66 | -1,03 | 0,42 | 0,49 | 851 |
| AA282404 | 0,02 | 0,07 | -0,29 | 0,46 | 0,58 | 852 |
| AI023336 | 0,00 | 0,24 | -0,13 | 0,28 | 0,27 | 853 |
| NM_001964 | 0,02 | -0,63 | -0,99 | 0,56 | 0,53 | 854 |
| N35603 | 0,04 | -0,51 | -0,87 | 0,51 | 0,67 | 855 |
| AI632210 | 0,00 | 0,35 | -0,01 | 0,60 | 0,26 | 856 |
| AA156454 | 0,00 | 0,37 | 0,01 | 0,34 | 0,35 | 857 |
| AA620836 | 0,02 | 0,24 | -0,12 | 0,51 | 0,55 | 858 |
| NM_020530 | 0,00 | 0,37 | 0,01 | 0,44 | 0,30 | 859 |
| AA928277 | 0,00 | -0,10 | -0,46 | 0,34 | 0,36 | 860 |
| NM_001559 | 0,04 | 0,37 | 0,01 | 0,73 | 0,50 | 861 |
| AA401691 | 0,00 | -0,08 | -0,44 | 0,39 | 0,38 | 862 |
| NM_015991 | 0,00 | 0,01 | -0,34 | 0,46 | 0,33 | 863 |
| N80764 | 0,00 | -0,08 | -0,43 | 0,33 | 0,43 | 864 |
| L34657 | 0,00 | 0,12 | -0,23 | 0,31 | 0,34 | 865 |
| H98244 | 0,00 | 0,24 | -0,11 | 0,39 | 0,35 | 866 |
| AA894523 | 0,00 | -0,24 | -0,59 | 0,23 | 0,29 | 867 |
| NM_013261.1 | 0,00 | 0,08 | -0,26 | 0,32 | 0,37 | 868 |
| H02254 | 0,01 | -0,39 | -0,73 | 0,40 | 0,45 | 869 |
| NM_003781.2 | 0,01 | -0,64 | -0,98 | 0,50 | 0,36 | 870 |
| NM_001243 | 0,05 | 0,78 | 0,44 | 0,51 | 0,66 | 871 |
| AA442897 | 0,01 | -0,44 | -0,78 | 0,32 | 0,46 | 872 |
| T85314 | 0,01 | -0.29 | -0,63 | 0,46 | 0,43 | 873 |
| AI658519 | 0,05 | 0,50 | 0,16 | 0,70 | 0,50 | 874 |
| AI207975 | 0,00 | -0,28 | -0,62 | 0,37 | 0,30 | 875 |
| AI536602 | 0,00 | 0,28 | -0,06 | 0,47 | 0,33 | 876 |
| NM_001541.1 | 0,00 | 0,50 | 0,16 | 0,38 | 0,27 | 877 |
| AA992540 | 0,00 | 0,14 | -0,19 | 0,31 | 0,32 | 878 |
| Z22971 | 0,01 | 0,62 | 0,29 | 0,51 | 0,39 | 879 |
| AI560847 | 0,00 | 0,36 | 0,03 | 0,23 | 0,28 | 880 |
| XM_008346 | 0,04 | 0,40 | 0,07 | 0,59 | 0,54 | 881 |
| AA015795 | 0,02 | -0,36 | -0,69 | 0,57 | 0,42 | 882 |
| R00742 | 0,00 | 0,37 | 0,04 | 0,34 | 0,33 | 883 |
| H16774 | 0,00 | 0,02 | -0,31 | 0,33 | 0,24 | 884 |
| R51373 | 0,00 | 0,15 | -0,18 | 0,31 | 0,24 | 885 |
| AI479659 | 0,00 | 0,18 | -0,14 | 0,34 | 0,29 | 886 |
| W58195 | 0,00 | -0,06 | -0,39 | 0,27 | 0,39 | 887 |
| NM_004437.1 | 0,05 | 1,06 | 0,73 | 0,47 | 0,65 | 888 |
| AA479357 | 0,00 | 0,18 | -0,15 | 0,30 | 0,21 | 889 |
| AI423518 | 0,00 | -0,25 | -0,57 | 0,29 | 0,40 | 890 |
| NM 002750 | 0,01 | -0,52 | -0,85 | 0,36 | 0,44 | 891 |
| R26444 | 0,00 | 0,00 | -0,32 | 0,27 | 0,36 | 892 |
| AA136071 | 0,00 | 0,04 | -0,29 | 0,25 | 0,34 | 893 |
| AI554459 | 0,00 | -0,02 | -0,34 | 0,39 | 0,35 | 894 |
| N51537 | 0,02 | 0,89 | 0,57 | 0,45 | 0,49 | 895 |
| NM_006068 | 0,00 | 0,62 | 0,30 | 0,35 | 0,39 | 896 |
| NM_016184 | 0,03 | 0,61 | 0,29 | 0,49 | 0,52 | 897 |
| NM 000586 | 0,03 | 0.03 | -0,29 | 0,40 | 0,54 | 898 |
| NM_003102.1 | 0,01 | -0,39 | -0,71 | 0,49 | 0,43 | 899 |
| AI264774 | 0,00 | -0,11 | -0,43 | 0,20 | 0.44 | 900 |
| N90536 | 0,01 | -0,45 | -0,77 | 0,30 | 0,45 | 901 |
| AA404342 | 0,00 | -0,29 | -0,61 | 0,36 | 0,36 | 902 |
| AI373525 | 0,00 | -0,16 | -0,48 | 0,30 | 0,25 | 903 |
| AI579907 | 0,00 | 0,07 | -0,25 | 0,38 | 0,25 | 904 |
| AA279410 | 0,00 | 0,11 | -0,21 | 0,33 | 0.26 | 905 |
| XM 038308 | 0,04 | 0,35 | 0,03 | 0.51 | 0,54 | 906 |
| NM 000879 | 0,02 | -0,01 | -0,33 | 0,37 | 0,52 | 907 |
| NM 001078) | 0,00 | 0,38 | 0,07 | 0,38 | 0,32 | 908 |
| AA781411 | 0,00 | -0,24 | -0,55 | 0,23 | 0,37 | 909 |
| R07171 | 0.00 | -0,16 | -0,48 | 0,34 | 0,37 | 910 |
| AA136273 | 0,00 | -0,10 | -0,41 | 0,26 | 0,32 | 911 |
| AI565469 | 0.01 | -0,06 | -0,37 | 0,32 | 0,41 | 912 |
| AI799767 | 0.00 | -0,12 | -0,44 | 0,35 | 0,36 | 913 |
| AI889554 | 0.00 | -0,08 | -0,39 | 0,34 | 0,36 | 914 |
| AA410301 | 0.01 | 0,77 | 0,46 | 0,35 | 0,42 | 915 |
| AA995114 | 0.04 | 1,09 | 0,79 | 0,67 | 0,40 | 916 |
| AI694444 | 0.00 | -0,40 | -0,71 | 0,26 | 0.35 | 917 |
| T98940 | 0.00 | 0,05 | -0,26 | 0,45 | 0,27 | 918 |
| R16722 | 0.00 | 0,07 | -0,23 | 0,42 | 0.23 | 919 |
| H05436 | 0.00 | 0,40 | 0,10 | 0,34 | 0,33 | 920 |
| R42778 | 0.01 | 0,39 | 0,09 | 0,45 | 0.33 | 921 |
| AI378275 | 0.00 | -0,02 | -0,33 | 0,29 | 0.40 | 922 |
| XM 083833 | 0,03 | 0.50 | 0,20 | 0,57 | 0,39 | 923 |
| R94894 | 0,03 | 1,00 | 0,70 | 0,35 | 0,55 | 924 |
| H15677 | 0,01 | -0,24 | -0,54 | 0,34 | 0,45 | 925 |
| AI625523 | 0,04 | 0,75 | 0,45 | 0,47 | 0,51 | 926 |
| AI627286 | 0,00 | 0,03 | -0,27 | 0,35 | 0,26 | 927 |
| NM 003807 | 0,01 | 0,08 | -0,22 | 0,35 | 0,42 | 928 |
| NM 002757 | 0,02 | 0,00 | -0,30 | 0,50 | 0,41 | 929 |
| XM 008411 | 0,02 | -0,47 | -0,77 | 0,31 | 0,51 | 930 |
| AI379294 | 0,01 | -0,06 | -0,35 | 0,45 | 0,32 | 931 |
| AI824470 | 0,00 | -0,20 | -0.49 | 0,19 | 0,42 | 932 |
| N94525 | 0.00 | 0,15 | -0.14 | 0.26 | 0.28 | 933 |
| R38432 | 0,01 | -0,03 | -0,32 | 0,27 | 0,41 | 934 |
| NM_017436.2 | 0,02 | -0,44 | -0,74 | 0,42 | 0,44 | 935 |
| AA398968 | 0,00 | -0,03 | -0,33 | 0,37 | 0,35 | 936 |
| U15085 | 0,03 | -0,89 | -1,18 | 0,47 | 0,47 | 937 |
| AI734941 | 0,01 | -0,14 | -0,43 | 0,31 | 0,41 | 938 |
| AI819159 | 0,00 | 0,44 | 0,15 | 0,39 | 0,28 | 939 |
| AA426024 | 0,02 | -0,11 | -0,40 | 0,46 | 0,42 | 940 |
| AA435854 | 0,00 | -0,33 | -0,62 | 0,21 | 0,28 | 941 |
| NM_003264 | 0,00 | 0,28 | -0,01 | 0,30 | 0,38 | 942 |
| NM_001622.1 | 0,04 | 0,01 | -0,28 | 0,41 | 0,53 | 943 |
| AI828714 | 0,04 | -0,25 | -0,55 | 0,33 | 0,54 | 944 |
| NM 006610 | 0,00 | -0,04 | -0,33 | 0,23 | 0,30 | 945 |
| AI143013 | 0,00 | -0,04 | -0,33 | 0,38 | 0,31 | 946 |
| AA428992 | 0,01 | 0,50 | 0,21 | 0,48 | 0,24 | 947 |
| R40560 | 0,02 | 0,17 | -0,12 | 0,33 | 0,44 | 948 |
| AI203091 | 0,02 | -0,44 | -0,73 | 0,28 | 0,50 | 949 |
| T92041 | 0,00 | 0,07 | -0,22 | 0,28 | 0.22 | 950 |
| AA453794 | 0,00 | 0,20 | -0,09 | 0,22 | 0,29 | 951 |
| R05804 | 0,00 | 0,18 | -0,11 | 0,22 | 0,34 | 952 |
| AA453489 | 0,01 | -0,56 | -0,85 | 0,33 | 0,37 | 953 |
| NM 006664 | 0,00 | 0,67 | 0,39 | 0,30 | 0,35 | 954 |
| AA281330 | 0,03 | 0,76 | 0,48 | 0,57 | 0,38 | 955 |
| AA452139 | 0,00 | 0,08 | -0,20 | 0,31 | 0,24 | 956 |
| R43204 | 0,00 | 0,19 | -0,09 | 0,38 | 0,21 | 957 |
| NM 012340 | 0,01 | 0,05 | -0,24 | 0,36 | 0,40 | 958 |
| NM 004778 | 0,02 | 0,00 | -0,28 | 0,43 | 0,40 | 959 |
| AA490815 | 0,01 | 0,04 | -0,24 | 0,26 | 0,44 | 960 |
| NM 022740 | 0,00 | 0,47 | 0,19 | 0,30 | 0,31 | 961 |
| AI167874 | 0,01 | 0,33 | 0,05 | 0,41 | 0,33 | 962 |
| AA149968 | 0,00 | -0,09 | -0,37 | 0,28 | 0,27 | 963 |
| XM_058179 | 0,03 | -0,04 | -0,32 | 0,58 | 0,35 | 964 |
| R07502 | 0,00 | -0,42 | -0,70 | 0,33 | 0,31 | 965 |
| NM 000752 | 0,01 | -0,27 | -0,56 | 0,48 | 0,29 | 966 |
| XM 003529 | 0,01 | 0,22 | -0,06 | 0,42 | 0,38 | 967 |
| N64541 | 0,01 | 0,13 | -0,15 | 0,44 | 0,37 | 968 |
| NM 001054 | 0,01 | 0,18 | -0,10 | 0,32 | 0,40 | 969 |
| Al499407 | 0,00 | 0,00 | -0,28 | 0,30 | 0,27 | 970 |
| NM 020056 | 0,00 | -0,05 | -0,33 | 0,32 | 0,28 | 971 |
| AA004952 | 0,01 | -0,20 | -0,48 | 0,41 | 0,31 | 972 |
| AI624610 | 0,01 | 0,09 | -0,19 | 0,34 | 0,38 | 973 |
| AA421924 | 0,04 | 0,92 | 0,64 | 0,49 | 0,44 | 974 |
| Al732550 | 0,04 | 0,03 | -0,25 | 0,51 | 0,43 | 975 |
| Al374599 | 0,02 | -0,15 | -0,43 | 0,24 | 0,47 | 976 |
| Al582909 | 0,00 | 0,34 | 0,06 | 0,21 | 0,21 | 977 |
| AI554111 | 0,00 | 0,21 | -0,07 | 0,39 | 0,21 | 978 |
| NM_001734 | 0,00 | -0,21 | -0,49 | 0,21 | 0,37 | 979 |
| AA810014 | 0,03 | 0,23 | -0,05 | 0,56 | 0,33 | 980 |
| AI373295 | 0,00 | 0,32 | 0,05 | 0,31 | 0,23 | 981 |
| XM_048555 | 0,01 | -0,20 | -0,48 | 0,38 | 0,34 | 982 |
| AA435627 | 0,00 | 0,15 | -0,13 | 0,31 | 0,26 | 983 |
| T95815 | 0,00 | 0,55 | 0,27 | 0,33 | 0,32 | 984 |
| AA426030 | 0,03 | -0,14 | -0,42 | 0,40 | 0,42 | 985 |
| AI720051 | 0,01 | -0,29 | -0,56 | 0,30 | 0,43 | 986 |
| AI278521 | 0,01 | -0,50 | -0,77 | 0,39 | 0,34 | 987 |
| N93236 | 0,01 | 0,38 | 0,10 | 0,38 | 0,34 | 988 |
| NM_015645 | 0,03 | -0,28 | -0,55 | 0,44 | 0,43 | 989 |
| AI671360 | 0,00 | 0,22 | -0,05 | 0,28 | 0,28 | 990 |
| T83666 | 0,00 | 0,13 | -0,14 | 0,36 | 0,21 | 991 |
| W02063 | 0,00 | -0,02 | -0,30 | 0,31 | 0,31 | 992 |
| AI659563 | 0,00 | 0,01 | -0,26 | 0,27 | 0,21 | 993 |
| NM_139046 | 0,02 | -0,47 | -0,74 | 0,35 | 0,45 | 994 |
| AA155745 | 0,00 | 0,00 | -0,27 | 0,31 | 0,26 | 995 |
| H40035 | 0,01 | -0,32 | -0,59 | 0,28 | 0,38 | 996 |
| AA101379 | 0,00 | 0,26 | -0,02 | 0,35 | 0,31 | 997 |
| H16790 | 0,00 | 0,22 | -0,05 | 0,37 | 0,28 | 998 |
| AA011511 | 0,02 | -0,29 | -0,55 | 0,32 | 0,41 | 999 |
| AA746495 | 0,05 | 0,17 | -0,10 | 0,56 | 0,39 | 1000 |
| AA845015 | 0,00 | -0,04 | -0,30 | 0,34 | 0,26 | 1001 |
| NM_138636 | 0,05 | 0,51 | 0,24 | 0,39 | 0,52 | 1002 |
| NM_033358 | 0,01 | 0,50 | 0,24 | 0,37 | 0,37 | 1003 |
| AI650349 | 0,02 | -0,13 | -0,39 | 0,40 | 0,41 | 1004 |
| NM_001764 | 0,01 | 0,33 | 0,06 | 0,46 | 0,20 | 1005 |
| XM_006447 | 0,03 | -0,53 | -0,80 | 0,49 | 0,39 | 1006 |
| R07185 | 0,00 | 0,12 | -0,14 | 0,34 | 0,22 | 1007 |
| AA187437 | 0,00 | -0,01 | -0,27 | 0,21 | 0,26 | 1008 |
| AI621365 | 0,00 | 0,25 | -0,02 | 0,34 | 0,28 | 1009 |
| NM_020205 | 0,03 | 0,16 | -0,10 | 0,29 | 0,48 | 1010 |
| AI888390 | 0,01 | -0,89 | -1,15 | 0,31 | 0,40 | 1011 |
| AI674699 | 0,01 | -0,09 | -0,35 | 0,34 | 0,37 | 1012 |
| AI620249 | 0,02 | 0,02 | -0,24 | 0,49 | 0,27 | 1013 |
| NM_033295 | 0,02 | -0,32 | -0,58 | 0,41 | 0,39 | 1014 |
| NM_015718.1 | 0,00 | -0,08 | -0,34 | 0,23 | 0,34 | 1015 |
| N73572 | 0,05 | 0,05 | -0,21 | 0,45 | 0,42 | 1016 |
| AI420037 | 0,02 | 0,04 | -0,22 | 0,46 | 0,31 | 1017 |
| AI684431 | 0,00 | 0,28 | 0,03 | 0,32 | 0,27 | 1018 |
| AA017263 | 0,00 | 0,11 | -0,14 | 0,38 | 0,25 | 1019 |
| R45118 | 0,01 | 0,16 | -0,10 | 0.32 | 0,33 | 1020 |
| AI267659 | 0,04 | 0,01 | -0,25 | 0,21 | 0,53 | 1021 |
| AA406083 | 0,03 | 0,00 | -0,26 | 0,38 | 0,41 | 1022 |
| W48664 | 0,00 | 0,21 | -0,05 | 0,31 | 0,22 | 1023 |
| AA514450 | 0,00 | -0,38 | -0,63 | 0,26 | 0,33 | 1024 |
| AI150305 | 0,00 | 0,30 | 0,04 | 0,23 | 0,32 | 1025 |
| AA481504 | 0,03 | -0,74 | -0,99 | 0,37 | 0,42 | 1026 |
| R44840 | 0,02 | 0,22 | -0,04 | 0,45 | 0,32 | 1027 |
| AI160757 | 0,00 | 0,21 | -0,05 | 0,29 | 0,29 | 1028 |
| AA040870 | 0,00 | 0,24 | -0,02 | 0,30 | 0,30 | 1029 |
| AI342905 | 0,02 | 0,49 | 0,24 | 0,43 | 0,35 | 1030 |
| N68463 | 0,05 | 0,09 | -0,16 | 0,46 | 0,43 | 1031 |
| AA398760 | 0,00 | 0,05 | -0,20 | 0,24 | 0,23 | 1032 |
| AI798514 | 0,00 | 0,26 | 0,00 | 0,30 | 0,25 | 1033 |
| AI081725 | 0,00 | 0,18 | -0,07 | 0,31 | 0,28 | 1034 |
| AI799385 | 0,03 | 0,44 | 0,19 | 0,45 | 0,37 | 1035 |
| AA897543 | 0,04 | -0,24 | -0,49 | 0,28 | 0,49 | 1036 |
| N79807 | 0,01 | 0,18 | -0,07 | 0,33 | 0,33 | 1037 |
| AI676097 | 0,05 | 0,21 | -0,04 | 0,57 | 0,32 | 1038 |
| R46372 | 0,01 | 0,02 | -0,23 | 0,28 | 0,37 | 1039 |
| AA448817 | 0,00 | 0,26 | 0,01 | 0,28 | 0,27 | 1040 |
| AI810161 | 0,01 | 0,09 | -0,16 | 0,31 | 0,38 | 1041 |
| H80437 | 0,00 | 0,18 | -0,07 | 0,23 | 0,29 | 1042 |
| AA443664 | 0,00 | -0,02 | -0,27 | 0,29 | 0,27 | 1043 |
| NM_002957.3 | 0,01 | -0,12 | -0,37 | 0,24 | 0,37 | 1044 |
| N69363 | 0,03 | -0,35 | -0,59 | 0,37 | 0,40 | 1045 |
| NM_000552.2 | 0,01 | -0,11 | -0,36 | 0,25 | 0,34 | 1046 |
| AA455080 | 0,01 | 0,08 | -0,16 | 0,34 | 0,28 | 1047 |
| W32272 | 0,00 | -0,25 | -0,50 | 0,26 | 0,30 | 1048 |
| H38087 | 0,04 | 0,76 | 0,51 | 0,34 | 0,47 | 1049 |
| AA504336 | 0,01 | 0,26 | 0,02 | 0,32 | 0,33 | 1050 |
| H04977 | 0,00 | 0,45 | 0,21 | 0,28 | 0,28 | 1051 |
| NM 002670 | 0,05 | 0,19 | -0,06 | 0,32 | 0,50 | 1052 |
| R09417 | 0,02 | -0,07 | -0,32 | 0,31 | 0,41 | 1053 |
| AA040057 | 0,02 | -0,05 | -0,29 | 0,35 | 0,37 | 1054 |
| AI263210 | 0,01 | -0,10 | -0,34 | 0,27 | 0,33 | 1055 |
| AI264626 | 0,01 | -0,12 | -0,37 | 0,33 | 0,29 | 1056 |
| AI478847 | 0,02 | 0,11 | -0,13 | 0,34 | 0,40 | 1057 |
| AI744042 | 0,03 | -0,37 | -0,61 | 0,51 | 0,27 | 1058 |
| AA682790 | 0,02 | 0,01 | -0,23 | 0,32 | 0,40 | 1059 |
| AA629051 | 0,01 | 0,28 | 0,04 | 0,32 | 0,29 | 1060 |
| AI560242 | 0,02 | -0,23 | -0,47 | 0,36 | 0,34 | 1061 |
| AA035428 | 0,01 | -0,14 | -0,38 | 0,26 | 0,32 | 1062 |
| NM_014326 | 0,02 | 0,11 | -0,13 | 0,52 | 0,15 | 1063 |
| AI632740 | 0,01 | -0,16 | -0,40 | 0,31 | 0,30 | 1064 |
| AI130878 | 0,01 | 0,27 | 0,03 | 0,32 | 0,31 | 1065 |
| AI933013 | 0,01 | 0,31 | 0,07 | 0,35 | 0,26 | 1066 |
| AI086719 | 0,01 | 0,00 | -0,24 | 0,37 | 0,24 | 1067 |
| R16568 | 0,03 | 0,10 | -0,14 | 0,24 | 0,46 | 1068 |
| AA009562 | 0,01 | -0,20 | -0,44 | 0,28 | 0,33 | 1069 |
| AI015069 | 0,01 | 0,04 | -0,20 | 0,32 | 0,34 | 1070 |
| AA291486 | 0,02 | -0,26 | -0,49 | 0,31 | 0,36 | 1071 |
| H65288 | 0,03 | -0,13 | -0,37 | 0,26 | 0,46 | 1072 |
| W86767 | 0,02 | 0,07 | -0,17 | 0,20 | 0,42 | 1073 |
| H65331 | 0,01 | 0,55 | 0,31 | 0,33 | 0,33 | 1074 |
| AA478985 | 0,04 | -0,12 | -0,36 | 0,20 | 0,51 | 1075 |
| H11274 | 0,02 | -0,02 | -0,26 | 0,28 | 0,40 | 1076 |
| AA044225 | 0,00 | -0,09 | -0,33 | 0,31 | 0,22 | 1077 |
| AI801415 | 0,00 | -0,08 | -0,32 | 0,32 | 0,23 | 1078 |
| AA846527 | 0,00 | -0,14 | -0,37 | 0,24 | 0,25 | 1079 |
| R56890 | 0,01 | -0,04 | -0,28 | 0,25 | 0,34 | 1080 |
| AI921525 | 0,03 | -0,06 | -0,29 | 0,36 | 0,40 | 1081 |
| AA405485 | 0,02 | 0,11 | -0,13 | 0,40 | 0,33 | 1082 |
| AA845635 | 0,00 | -0,03 | -0,26 | 0,31 | 0,26 | 1083 |
| AI150418 | 0,01 | 0,07 | -0,17 | 0,23 | 0,33 | 1084 |
| XM 049849 | 0,02 | 0,55 | 0,32 | 0,32 | 0,37 | 1085 |
| AA406573 | 0,00 | 0,20 | -0,03 | 0,33 | 0,23 | 1086 |
| AA043930 | 0,01 | -0,26 | -0,49 | 0,27 | 0,35 | 1087 |
| AI125496 | 0,01 | -0,30 | -0,53 | 0,29 | 0,33 | 1088 |
| AI654739 | 0,02 | -0,06 | -0,29 | 0,31 | 0,35 | 1089 |
| AA398320 | 0,01 | -0,32 | -0,56 | 0,37 | 0,30 | 1090 |
| NM 002155 | 0,04 | 0,48 | 0,25 | 0,36 | 0,43 | 1091 |
| AA505872 | 0,01 | 0,71 | 0,48 | 0,31 | 0,34 | 1092 |
| NM 016610 | 0,02 | 0,24 | 0,00 | 0,20 | 0,43 | 1093 |
| AA703200 | 0,00 | -0,13 | -0,36 | 0,26 | 0,29 | 1094 |
| R44493 | 0,00 | 0,04 | -0,19 | 0,24 | 0,23 | 1095 |
| XM_046575 | 0,04 | -0,14 | -0,38 | 0,40 | 0,40 | 1096 |
| AI275613 | 0,03 | 0,24 | 0,00 | 0,44 | 0,30 | 1097 |
| AI308602 | 0,04 | 0,19 | -0,05 | 0,38 | 0,40 | 1098 |
| R44328 | 0,01 | 0,24 | 0,01 | 0,30 | 0,30 | 1099 |
| R00206 | 0,00 | 0,07 | -0,16 | 0,23 | 0,31 | 1100 |
| NM_002456 | 0,01 | 0,02 | -0,21 | 0,37 | 0,25 | 1101 |
| AI699371 | 0,03 | -0,18 | -0,41 | 0,46 | 0,28 | 1102 |
| AA935135 | 0,03 | 0,21 | -0,02 | 0,41 | 0,33 | 1103 |
| AA702529 | 0,02 | 0,06 | -0,17 | 0,40 | 0,27 | 1104 |
| AI568023 | 0,02 | -0,19 | -0,42 | 0,36 | 0,30 | 1105 |
| NM_002768 | 0,01 | -0,65 | -0,88 | 0,27 | 0,31 | 1106 |
| AA687208 | 0,02 | -0,32 | -0,55 | 0,24 | 0,39 | 1107 |
| AI221524 | 0,04 | 0,47 | 0,25 | 0,49 | 0,31 | 1108 |
| AA813007 | 0,01 | 0,09 | -0,13 | 0,24 | 0,33 | 1109 |
| AA421326 | 0,02 | -0,33 | -0,55 | 0,28 | 0,38 | 1110 |
| AA922397 | 0,01 | 0,06 | -0,17 | 0,19 | 0,33 | 1111 |
| R51857 | 0,03 | 0,90 | 0,67 | 0,40 | 0,30 | 1112 |
| NM 006564 | 0,00 | -0,22 | -0,44 | 0,33 | 0,21 | 1113 |
| AA807376 | 0,01 | 0.39 | 0,17 | 0,31 | 0,25 | 1114 |
| AA812763 | 0,04 | -0,45 | -0,68 | 0,36 | 0,37 | 1115 |
| AA528169 | 0.02 | 0,34 | 0,12 | 0,37 | 0,32 | 1116 |
| A1804325 | 0,01 | -0,14 | -0,36 | 0,32 | 0,24 | 1117 |
| T70330 | 0,04 | -0,10 | -0,33 | 0,30 | 0,41 | 1118 |
| NM 001766 | 0,03 | 0,30 | 0,08 | 0,39 | 0,35 | 1119 |
| A1696956 | 0,01 | -0,12 | -0,34 | 0,40 | 0,23 | 1120 |
| A1459174 | 0,01 | -0,05 | -0,27 | 0,30 | 0,25 | 1121 |
| R35639 | 0,01 | -0,03 | -0,25 | 0,17 | 0,37 | 1122 |
| W69774 | 0,01 | -0,02 | -0,24 | 0,21 | 0,30 | 1123 |
| AA054265 | 0,05 | 0,37 | 0,15 | 0,40 | 0,38 | 1124 |
| A1382995 | 0,01 | 0,19 | -0,03 | 0,29 | 0,25 | 1125 |
| Al218303 | 0,01 | 0,00 | -0,22 | 0,31 | 0.26 | 1126 |
| A1624954 | 0,01 | -0,18 | -0,40 | 0,28 | 0,31 | 1127 |
| AA759254 | 0,05 | -0,08 | -0,30 | 0,49 | 0,29 | 1128 |
| Al682979 | 0,02 | 0,03 | -0,19 | 0,22 | 0,37 | 1129 |
| XM 001754 | 0.01 | 0,18 | -0,03 | 0,31 | 0,28 | 1130 |
| Al187401 | 0,00 | -0,01 | -0,23 | 0,21 | 0,22 | 1131 |
| AA452113 | 0,01 | 0,24 | 0,02 | 0,25 | 0,30 | 1132 |
| Al656210 | 0,04 | -0,48 | -0,70 | 0,30 | 0,40 | 1133 |
| N29999 | 0,01 | 0,21 | 0,00 | 0,22 | 0,34 | 1134 |
| N68557 | 0,01 | 0,00 | -0,21 | 0,19 | 0,32 | 1135 |
| Al689672 | 0,02 | -0,08 | -0,29 | 0,42 | 0,19 | 1136 |
| AA730310 | 0,00 | -0,07 | -0,28 | 0,25 | 0,22 | 1137 |
| Al431324 | 0,01 | -0,20 | -0,42 | 0,39 | 0,22 | 1138 |
| NM 000066 | 0,04 | -0,11 | -0,32 | 0,31 | 0,40 | 1139 |
| XM_034219 | 0,01 | 0,01 | -0,21 | 0,30 | 0,29 | 1140 |
| R43258 | 0,04 | 0,27 | 0,05 | 0,49 | 0,23 | 1141 |
| Al431293 | 0,00 | 0,07 | -0,15 | 0,25 | 0,22 | 1142 |
| R80259 | 0,04 | -0,49 | -0,70 | 0,22 | 0,38 | 1143 |
| Al126520 | 0,00 | 0,13 | -0,08 | 0,22 | 0,21 | 1144 |
| AA937226 | 0,00 | 0,02 | -0,19 | 0,25 | 0,26 | 1145 |
| Al191762 | 0,03 | -0,22 | -0,43 | 0,30 | 0,36 | 1146 |
| AA400470 | 0,00 | -0,10 | 0,31 | 0,34 | 0,17 | 1147 |
| NM_000063 | 0,01 | -0,17 | -0,38 | 0,29 | 0,23 | 1148 |
| H73962 | 0,01 | -0,11 | -0,32 | 0,22 | 0,30 | 1149 |
| AA626313 | 0,01 | -0,06 | -0,27 | 0,23 | 0,30 | 1150 |
| Al553630 | 0,03 | 0,13 | -0,08 | 0,36 | 0,31 | 1151 |
| NM 000257.1 | 0,01 | 0,37 | 0,16 | 0,29 | 0,25 | 1152 |
| N68456 | 0,03 | 0,33 | 0,12 | 0,27 | 0,36 | 1153 |
| XM_054837 | 0,01 | 0,24 | 0,04 | 0,24 | 0,27 | 1154 |
| AI696558 | 0,04 | -0,49 | -0,70 | 0,38 | 0,33 | 1155 |
| AI299876 | 0,05 | 0,03 | -0,18 | 0,36 | 0,37 | 1156 |
| NM_006378 | 0,03 | 0,65 | 0,44 | 0,28 | 0,36 | 1157 |
| AI376955 | 0,02 | -0,56 | -0,76 | 0,31 | 0,33 | 1158 |
| AA025573 | 0,01 | -0,24 | -0,45 | 0,33 | 0,25 | 1159 |
| T99196 | 0,02 | 0,14 | -0,07 | 0,34 | 0,29 | 1160 |
| XM_005637 | 0,05 | 0,25 | 0,05 | 0,19 | 0,45 | 1161 |
| AI597729 | 0,04 | -0,01 | -0,21 | 0,24 | 0,41 | 1162 |
| H78135 | 0,02 | 0,04 | -0,17 | 0,29 | 0,33 | 1163 |
| AI695029 | 0,01 | 0,04 | -0,16 | 0,27 | 0,25 | 1164 |
| AA004279 | 0,02 | -0,18 | -0,39 | 0,21 | 0,34 | 1165 |
| AA844020 | 0,03 | 0,33 | 0,12 | 0,30 | 0,33 | 1166 |
| AI332536 | 0,00 | -0,12 | -0,33 | 0,20 | 0,18 | 1167 |
| AI383368 | 0,03 | -0,40 | -0,61 | 0,21 | 0,38 | 1168 |
| AA423883 | 0,00 | -0,06 | -0,26 | 0,17 | 0,28 | 1169 |
| R36006 | 0,02 | -0,06 | -0,26 | 0,30 | 0,29 | 1170 |
| AI911837 | 0,02 | -0,05 | -0,26 | 0,30 | 0,31 | 1171 |
| AI696820 | 0,03 | -0,37 | -0,57 | 0,32 | 0,34 | 1172 |
| H30516 | 0,02 | -0,17 | -0,37 | 0,22 | 0,34 | 1173 |
| AI926561 | 0,01 | 0,02 | -0,18 | 0,37 | 0,20 | 1174 |
| H61449 | 0,02 | -0,25 | -0,45 | 0,24 | 0,32 | 1175 |
| AA410338 | 0,02 | -0,18 | -0,38 | 0,37 | 0,26 | 1176 |
| AA485229 | 0,00 | 0,05 | -0,15 | 0,18 | 0,18 | 1177 |
| AA044828 | 0,01 | -0,01 | -0,21 | 0,22 | 0,31 | 1178 |
| R07278 | 0,03 | 0,00 | -0,20 | 0,15 | 0,39 | 1179 |
| AI687656 | 0,02 | -0,22 | -0,42 | 0,28 | 0,28 | 1180 |
| AI912316 | 0,03 | 0,21 | 0,01 | 0,42 | 0,24 | 1181 |
| AA017301 | 0,00 | -0,07 | -0,27 | 0,18 | 0,26 | 1182 |
| AA059314 | 0,05 | 0,13 | -0,07 | 0,26 | 0,40 | 1183 |
| NM_024302.2 | 0,04 | 0,20 | 0,00 | 0,29 | 0,35 | 1184 |
| AA446463 | 0,02 | -0,15 | -0,34 | 0,29 | 0,29 | 1185 |
| NM_002747 | 0,01 | 0,19 | -0,01 | 0,24 | 0,24 | 1186 |
| AA446316 | 0,02 | 0,03 | -0,17 | 0,30 | 0,29 | 1187 |
| NM_052813) | 0,05 | -0,22 | -0,42 | 0,39 | 0,30 | 1188 |
| AA731532 | 0,00 | -0,24 | -0,43 | 0,18 | 0,24 | 1189 |
| R00307 | 0,04 | 0,16 | -0,03 | 0,45 | 0,20 | 1190 |
| AI924296 | 0,03 | -0,08 | -0,28 | 0,19 | 0,36 | 1191 |
| AI017741 | 0,01 | 0,07 | -0,12 | 0,29 | 0,21 | 1192 |
| AI619681 | 0,01 | -0,18 | -0,37 | 0,17 | 0,29 | 1193 |
| AA400967 | 0,01 | 0,25 | 0,06 | 0,30 | 0,22 | 1194 |
| NM_000680.1 | 0,01 | 0,28 | 0,09 | 0.20 | 0,28 | 1195 |
| AI732878 | 0,00 | -0,09 | -0,28 | 0,16 | 0,16 | 1196 |
| XM_006454 | 0,02 | -0,08 | -0,207 | 0,34 | 0,24 | 1197 |
| AI688916 | 0,03 | 0,02 | -0,17 | 0,32 | 0,29 | 1198 |
| T79834 | 0,01 | 0,09 | -0,10 | 0,25 | 0,27 | 1199 |
| AI015693 | 0,01 | -0,01 | -0,20 | 0,20 | 0,27 | 1200 |
| R50755 | 0,00 | -0,01 | -0,20 | 0,19 | 0,19 | 1201 |
| W44337 | 0,04 | 0,05 | -0,13 | 0,18 | 0,39 | 1202 |
| H23267 | 0,03 | -0,37 | -0,55 | 0,26 | 0,31 | 1203 |
| AA101850 | 0,02 | -0,12 | -0,30 | 0,34 | 0,21 | 1204 |
| AI628322 | 0,05 | -0,03 | -0,22 | 0,37 | 0,28 | 1205 |
| R94207 | 0,02 | 0,13 | -0,06 | 0,26 | 0,28 | 1206 |
| NM_004347 | 0,03 | 0,32 | 0,14 | 0,35 | 0,27 | 1207 |
| AA960802 | 0,05 | 0,14 | -0,04 | 0,37 | 0,29 | 1208 |
| NM_052962 | 0,02 | -0,42 | -0,60 | 0,26 | 0,25 | 1209 |
| T91946 | 0,04 | 0,14 | -0,05 | 0,29 | 0,33 | 1210 |
| AA531564 | 0,04 | -0,14 | -0,32 | 0,37 | 0,26 | 1211 |
| R96155 | 0,01 | 0,00 | -0,18 | 0,28 | 0,21 | 1212 |
| AI825491 | 0,02 | -0,07 | -0,25 | 0,19 | 0,29 | 1213 |
| N53973 | 0,02 | 0,01 | -0,17 | 0,22 | 0,31 | 1214 |
| NM_001544 | 0,01 | 0,10 | -0,08 | 0,27 | 0,22 | 1215 |
| AA702731 | 0,00 | -0,16 | -0,34 | 0,19 | 0,23 | 1216 |
| AI554655 | 0,05 | -0,04 | -0,22 | 0,23 | 0,36 | 1217 |
| H17495 | 0,04 | 0,50 | 0,32 | 0,29 | 0,31 | 1218 |
| AI209185 | 0,02 | -0,24 | -0,42 | 0.16 | 0,31 | 1219 |
| AA031813 | 0,03 | -0,15 | -0,33 | 0,29 | 0,27 | 1220 |
| NM_004166 | 0,04 | -0,37 | -0,54 | 0,35 | 0,27 | 1221 |
| AA461044 | 0,02 | 0,06 | -0,11 | 0,21 | 0,31 | 1222 |
| N45328 | 0,05 | -0,12 | -0,29 | 0,32 | 0,30 | 1223 |
| N64446 | 0,03 | -0,24 | -0,42 | 0,24 | 0,32 | 1224 |
| A1633617 | 0,01 | -0,05 | -0,22 | 0,23 | 0,24 | 1225 |
| R45159 | 0,03 | 0,22 | 0,05 | 0,32 | 0,24 | 1226 |
| R60898 | 0,00 | 0,13 | -0,04 | 0,18 | 0,17 | 1227 |
| AI621170 | 0,03 | -0,05 | -0,22 | 0,30 | 0,27 | 1228 |
| N99049 | 0,01 | 0,16 | -0,01 | 0,31 | 0,19 | 1229 |
| H18651 | 0,01 | 0,19 | 0,02 | 0,24 | 0,22 | 1230 |
| AA568582 | 0,04 | 0,02 | -0,15 | 0,30 | 0,28 | 1231 |
| AA026871 | 0,03 | -0,02 | -0,19 | 0,37 | 0,20 | 1232 |
| Al559626 | 0,01 | -0,11 | -0,28 | 0,23 | 0,21 | 1233 |
| AA443545 | 0,03 | 0,46 | 0,29 | 0,27 | 0,28 | 1234 |
| R43339 | 0,04 | 0,22 | 0,06 | 0,36 | 0,23 | 1235 |
| AA007369 | 0,04 | -0,16 | -0,33 | 0,28 | 0,30 | 1236 |
| AA960982 | 0,01 | 0,25 | 0,08 | 0,26 | 0,22 | 1237 |
| AA481399 | 0,01 | 0,01 | -0,16 | 0,30 | 0,18 | 1238 |
| AA280005 | 0,02 | -0,17 | -0,34 | 0,23 | 0.26 | 1239 |
| NM_005666 | 0,01 | 0,36 | 0,19 | 0,26 | 0,20 | 1240 |
| NM_000491 | 0,03 | 0,08 | -0,09 | 0,32 | 0,24 | 1241 |
| AA844053 | 0,03 | -0,12 | -0,28 | 0,22 | 0,26 | 1242 |
| R49384 | 0,01 | -0,05 | -0,22 | 0,24 | 0,21 | 1243 |
| AI698289 | 0,01 | -0,16 | -0,33 | 0,23 | 0,21 | 1244 |
| AI680467 | 0,04 | -0,09 | -0,26 | 0,23 | 0,31 | 1245 |
| M90391 | 0,03 | -0,11 | -0,28 | 0,27 | 0,23 | 1246 |
| AF218727 | 0,05 | 0,18 | 0,02 | 0,25 | 0,31 | 1247 |
| H22946 | 0,04 | -0,44 | -0,60 | 0,27 | 0,29 | 1248 |
| N49285 | 0,03 | -0,51 | -0,67 | 0,23 | 0,28 | 1249 |
| N74903 | 0,01 | 0,17 | 0,01 | 0,21 | 0,22 | 1250 |
| NM_001066.2 | 0,04 | 0,14 | -0,02 | 0,23 | 0,30 | 1251 |
| NM_021805 | 0,02 | 0,05 | -0,11 | 0,29 | 0,21 | 1252 |
| NM_004590 | 0,04 | 0,26 | 0,10 | 0,27 | 0,27 | 1253 |
| AA482392 | 0,01 | -0,19 | -0,35 | 0,25 | 0,20 | 1254 |
| AA131826 | 0,01 | -0,04 | -0,20 | 0,26 | 0,17 | 1255 |
| AA947111 | 0,02 | 0,07 | -0,09 | 0,17 | 0,27 | 1256 |
| AI159796 | 0,04 | -0,13 | -0,28 | 0,20 | 0,28 | 1257 |
| AF086537 | 0,05 | 0,15 | -0,01 | 0,32 | 0,24 | 1258 |
| AI147932 | 0,00 | 0,13 | -0,03 | 0,23 | 0,16 | 1259 |
| AA460956 | 0,04 | 0,11 | -0,04 | 0,30 | 0,24 | 1260 |
| AA398249 | 0,03 | -0,11 | -0,27 | 0,23 | 0,26 | 1261 |
| H08161 | 0,04 | -0,23 | -0,39 | 0,23 | 0,28 | 1262 |
| AA281734 | 0,03 | -0,12 | -0,28 | 0,31 | 0,19 | 1263 |
| AA628488 | 0,04 | -0,18 | -0,33 | 0,20 | 0,30 | 1264 |
| AA430519 | 0,04 | -0,06 | -0,21 | 0,22 | 0,26 | 1265 |
| AA468113 | 0,05 | -0,16 | -0,31 | 0,29 | 0,25 | 1266 |
| AI424466 | 0,04 | 0,06 | -0,10 | 0,26 | 0,24 | 1267 |
| AI190760 | 0,04 | 0,04 | -0,11 | 0,29 | 0,23 | 1268 |
| N89992 | 0,01 | -0,06 | -0,21 | 0,23 | 0,18 | 1269 |
| AA046092 | 0,01 | 0,10 | -0,05 | 0,16 | 0,21 | 1270 |
| W35358 | 0,02 | 0,05 | -0,10 | 0,22 | 0,20 | 1271 |
| AA398341 | 0,04 | -0,19 | -0,33 | 0,27 | 0,23 | 1272 |
| H01969 | 0,05 | -0,09 | -0,24 | 0,32 | 0,20 | 1273 |
| AA970008 | 0,05 | -0,34 | -0,48 | 0,32 | 0,21 | 1274 |
| R89846 | 0,01 | 0,14 | -0,01 | 0,20 | 0,20 | 1275 |
| H18639 | 0,04 | 0,13 | -0,02 | 0,26 | 0,24 | 1276 |
| Al016342 | 0,02 | 0,02 | -0,12 | 0,18 | 0,22 | 1277 |
| NM_002184 | 0,04 | -0,23 | -0,37 | 0,17 | 0,28 | 1278 |
| NM_001643.1 | 0,03 | 0,13 | -0,01 | 0,19 | 0,26 | 1279 |
| AA280029 | 0,04 | -0,14 | -0,28 | 0,28 | 0,22 | 1280 |
| AA927949 | 0,00 | 0,17 | 0,02 | 0,16 | 0,14 | 1281 |
| AA625552 | 0,04 | 0,05 | -0,09 | 0,28 | 0,20 | 1282 |
| AA458912 | 0,03 | -0,23 | -0,37 | 0,24 | 0,23 | 1283 |
| AI188025 | 0,02 | 0,29 | 0,15 | 0,21 | 0,22 | 1284 |
| XM_007417 | 0,02 | 0,00 | -0,14 | 0,21 | 0,19 | 1285 |
| AA019529 | 0,03 | -0,29 | -0,42 | 0,22 | 0,22 | 1286 |
| AA401542 | 0,04 | -0.09 | -0,22 | 0,17 | 0,25 | 1287 |
| AI478746 | 0,04 | 0,00 | -0,13 | 0,23 | 0,22 | 1288 |
| AA291522 | 0,01 | -0,33 | -0,47 | 0,14 | 0,22 | 1289 |
| AI493122 | 0,05 | 0,16 | 0,03 | 0,25 | 0,22 | 1290 |
| AI203665 | 0,02 | 0,11 | -0,02 | 0,22 | 0,18 | 1291 |
| R74060 | 0,05 | -0,15 | -0,28 | 0,20 | 0,24 | 1292 |
| AI185721 | 0,04 | -0,25 | -0,37 | 0,22 | 0,19 | 1293 |
| AA437106 | 0,05 | 0,10 | -0,02 | 0,23 | 0,20 | 1294 |
| NM 139208 | 0,04 | -0,07 | -0,18 | 0,22 | 0,20 | 1295 |
| AI922221 | 0,05 | -0,02 | -0.14 | 0,20 | 0,20 | 1296 |
| AA412418 | 0,05 | -0,26 | -0.37 | 0,19 | 0,18 | 1297 |

Diese in Tabelle 2 und 3 charakteristischen Veränderungen sind für die erfindungsgemäße Verwendung gemäß Anspruch 1 ausnutzbar.

Die in den Tabellen 2 und 3 aufgeführten GenBank Accession Nummern (Internet-Zugang über http://www.ncbi.nlm.nih.gov/) der einzelnen Sequenzen sind in dem dieser Anmeldung angefügten Sequenzprotokol im Einzelnen jeweils einer Sequenz ID (Sequenz ID: 1 bis zur Sequenz ID: 1297) zugeordnet.

### Referenzen

1. Natanson C (1997) Anti-inflammatory therapies to treat sepsis and septic shock: A reassessment. Crit Care Med 25: 1095-1099
2. Geiger K (1995) Frühparameter für Multiorgandysfunktionssyndrom in Hartenauer U (ed.) Sepsis in der Frühphase München MMV Medizin Verlag 19-25
3. Knaus WA , Draper EA, Wagner DP, Zimmermann JE (1985) Prognosis in acute organ-system failure. Ann Surg 202: 658-693
4. Goris RI, Bockhorst TP , Nuytinck JKS (1995) Mulitiple organ failure. Arch Surg 120:1109-1115
5. Vincent JL, Moreno R, Takala J, et al. (1996) The SOFA (Sepsis-related Organ Failure Assessment) score to describe organ dysfunction/failure. On behalf of the Working Group on Sepsis-Related Problems of the European Society of Intensive Care Medicine, Intensive Care Med. Jul 22(7):707-10.
6. Pfeiffer L, Ehrhardt N, Kretschmar R, et al. (1996) Endotoxinämie und Multiorganversagen nach Polytrauma. Anaesthesiol Reanimat 21: 91-96
7. Schlag G, RedI H (1993) Organ in shock, early organ failure, late organ failure., in Schlag G and RedI H (eds.) Pathophysiology of shock, sepsis, and organ failure Berlin Heidelberg Springer-Verlag, 1-4
8. Bone RC, Balk RA, Cerra FB, et al. (1992) The ACCP/SCCM Consensus Conference Committee (1992) Definitions for Sepsis and organ failure and guidelines for the use of innovative therapies in Sepsis. Chest 101:1656-1662; und Crit Care Med 1992; 20: 864-874.
9. Levy MM, Fink M, Marshall JC, et al. (2003) For the International Sepsis Definitions Conference: 2001 SCCM/ESICM/ACCP/ATS/SIS International Sepsis Definitions Conference. Crit Care Med. Apr;31 (4):1250-6
10. http://chirinn.klinikum.uni-muenchen.de/forschung/for 01_14 04.html, Stand Otober 2004, modifiziert
11.Marik PE. (1993) Gastric intramucosal pH. A better predictor of multiorgan dysfuction syndrom and death than oxygen derived variables in patients with sepsis. CHEST 104:, 225-229
12. Bernardin G , Pradier C, Tiger F, et al. (1996) Blood pressure and arterial lactate level are early indicators of short-term survival in human septic shock. Intensiv Care Med 22: 17-25;
13. Marecaux G, Pinsky MR, Dupont E, et al. (1996) Blood lactate levels are better prognostic indicators than TNF and IL-6 levels in patients with septic shock. Intensiv Care Med 22: 404-408
14. Duswald KH , Jochum M , Schramm W , Fritz H (1985) Released granulocytic elastase: an indicator of pathobiochemical alterations in septicemia after abdominal surgery. Surgery 98: 892-899
15. Nuytinck JKS, Goris RI, Redl H, et al. (1986) Posttraumatic complications and inflammatory mediators. Arch Surg 121: 886-890
16.Nast-Kolb D, Jochum M, Waydlas C, et al. (1991) Die Wertigkeit biochemischer Faktoren beim Polytrauma. Hefte Unfallheilkunde 215: 215
17. Hack CE, de Groot ER , Felt-Bersma RJ , et al. (1989): Increased plasma levels of interleukin-6 in sepsis" Blood 74: 1704-1710
18. Patel RT, Deen KI, Youngs D, et al. (1994) Interleukin 6 is a prognostic indicator of outcome in severe intra-abdominal sepsis. Br J Surg 81:1306-1308
19. Southern EM (1974) An improved method for transferring nucleotides from electrophoresis strips to thin layers of ion-exchange cellulose. Anal Biochem 62:317-318
20. Gillespie D, Spiegelman S (1965) A quantitative assay for DNA-RNA hybrids with DNA immobilized on a membrane. J Mol Biol 12:829-842
21. Lennon GG, Lehrach H (1991) Hybridization analyses of arrayed cDNA libraries. Trends Genet 7: 314-317
22. Kafatos FC, Jones CW, Efstratiadis A (1979) Determination of nucleic acid sequence homologies and relative concentrations by a dot hybridization procedure. Nucl Acid Res 7:1541-1552
23.Fodor SP, Read JL, Pirrung MC, Stryer L, Lu AT, Solas D (1991) Lightdirected, spatially addressable parallel chemical synthesis. Science 251:767-773
24. Pease AC, Solas D, Sullivan EJ, Cronin MT, Holmes CP, Fodor SP (1994) Light-generated oligonucleotide arrays for rapid DNA sequence analysis. Proc Natl Acad Sci USA 91:5022-5026
25. Schena M, Shalon D, Davis RW, Brown PO (1995) Quantitative monitoring of gene expression patterns with a complementary DNA microarray. Science 270:467-470
26.Golub TR, Slonim DK, Tamayo P, et al. (1999) Molecular classification of cancer: class discovery and class prediction by gene expression monitoring. Science 286:531-537
27. Alizadeh AA, Eisen MB, Davis RE, et al. (2000) Distinct types of diffuse large B-cell lymphoma identified by gene expression profiling. Nature 403:503-51
28. Feezor RJ, Baker HV, Xiao W, et al. (2004) Genomic and Proteomic Determinants of outcome in patients undergoing thoracoabdominal aortic aneurysm repair. Journal of Immunology 172 (11): 7103-7109
29.Cobb JP, Laramie JM, Stormo GD et al. (2002) Sepsis gene expression profiling: Murine splenic compared with hepatic responses determined by using complementary DNA microarrays. Crit Care Med 30(12):2711-2720
30.Tsukahara Y, Lian Z, Zhang X et al. (2003) Gene Expression in Human Neutrophils During Activation and Priming by Bacterial Lipopolysaccharide. J Cell Biochem 89: 848-861
31. Huber W, Heydebreck A, Sueltmann H, et al. (2003) Parameter estimation for the calibration and variance stabilization of microarray data. Stat. Appl. in Gen. and Mol. Biol. Vol. 2, Issue 1, Article 3

## Patentansprüche

1. Verfahren zur in vitro Messung von Genexpressionsprofilen unter Verwendung spezifischer Gene ausgewählt aus der Gruppe bestehend aus: SEQ-ID No. 1 bis SEQ-ID No. 1297 sowie Genfragmenten davon mit wenigstens 20-2000 Nukleotiden, **dadurch gekennzeichnet, dass** die Genexpression einer Mehrzahl von Genen aus Proben von Patienten bestimmt wird, um das Vorliegen einer nichtinfektiösen oder infektiösen Ursache eines Multiorganversagens festzustellen.

2. Verfahren nach Anspruch 1, **dadurch gekennzeichnet, dass** die Genfragmente 20 - 200, vorzugsweise 20-80 Nukleotide umfassen.

3. Verfahren nach Anspruch 1 oder 2, **dadurch gekennzeichnet, dass** wenigstens 2 bis 100 unterschiedliche Gene und/oder Genfragmente verwendet werden.

4. Verfahren nach einem der Ansprüche 1 oder 2, **dadurch gekennzeichnet, daß** wenigstens 200 unterschiedliche Gene und/oder Genfragmente verwendet werden.

5. Verfahren nach einem der Ansprüche 1 oder 2 **dadurch gekennzeichnet, daß** wenigstens 200 bis 500 unterschiedliche Gene und/oder Genfragmente verwendet werden.

6. Verfahren nach einem der Ansprüche 1 oder 2 **dadurch gekennzeichnet, daß** wenigstens 500 bis 1000 unterschiedliche Gene und/oder Genfragmente verwendet werden.

7. Verfahren nach einem der Ansprüche 1 oder 2 **dadurch gekennzeichnet, daß** wenigstens 1000 bis 2000 unterschiedliche Gene und/oder Genfragmente verwendet werden.

8. Verfahren nach Anspruch 1 bis 7, **dadurch gekennzeichnet, daß** die Genexpression mittels Hybridisierungsverfahren bestimmt wird.

9. Verfahren nach Anspruch 8, **dadurch gekennzeichnet, dass** die Genexpression mittels Microarrays bestimmt wird.

10. Verfahren nach einem der Ansprüche 1 bis 9, **dadurch gekennzeichnet, dass** die Genexpression durch Hybridisierungs-unabhängige Verfahren, insbesondere enzymatische und/oder chemische Hydrolyse und/oder Amplifikationsverfahren, vorzugsweise PCR, anschließende Quantifizierung der Nukleinsäuren und/oder von Derivaten und/oder Fragmenten derselben, bestimmt wird.

11. Verfahren nach einem der Ansprüche 1 bis 10, **dadurch gekennzeichnet, daß** die Probe ausgewählt wird aus: Körperflüssigkeiten, insbesondere Blut, Liquor, Urin, Ascitesflüssigkeit, Seminalflüssigkeit, Speichel, Punktat; Zellinhalt oder eine Mischung davon.

12. Verfahren nach einem der Ansprüche 1 bis 11, **dadurch gekennzeichnet, daß** Zellproben gegebenenfalls einer lytischen Behandlung unterzogen werden, um deren Zellinhalte freizusetzen.

## Claims

1. Method for in vitro measurement of gene expression profiles using specific genes selected from the group comprising: SEQ-ID No. 1 to SEQ-ID Bo. 1297 as well as gene fragments thereof with at least 20-2000 nucleotides, **characterised in that** the gene expression of a plurality of genes from samples from patients is determined in order to ascertain the presence of a non-infectious or infectious cause of a multi-organ failure.

2. Method according to Claim 1, **characterised in that** the gene fragments encompass 20 - 200, preferably 20 - 80 nucleotides.

3. Method according to Claim 1 or 2, **characterised in that** at least 2 to 100 different genes and/or gene fragments are used.

4. Method according to one of Claims 1 or 2, **characterised in that** at least 200 different genes and/or gene fragments are used.

5. Method according to one of Claims 1 or 2, **characterised in that** at least 200 to 500 different genes and/or gene fragments are used.

6. Method according to one of Claims 1 or 2, **characterised in that** at least 500 to 1000 different genes and/or gene fragments are used.

7. Method according to one of Claims 1 or 2, **characterised in that** at least 1000 to 2000 different genes and/or gene fragments are used.

8. Method according to Claim 1 to 7, **characterised in that** the gene expression is determined by means of hybridisation methods.

9. Method according to Claim 8, **characterised in that** the gene expression is determined by means of microassays.

10. Method according to one of Claims 1 to 9, **characterised in that** the gene expression is determined by hybridisation-independent methods, in particular enzymatic and/or chemical hydrolysis and/or amplification methods, preferably PCR, subsequent quantification of the nucleic acids and/or of derivatives and/or fragments of the same.

11. Method according to one of Claims 1 to 10, **characterised in that** the sample is selected from: Body fluids, in particular blood, liquor, urine, ascitic fluid, seminal fluid, saliva, punctate; cell content or a mixture thereof.

12. Method according to one of Claims 1 to 11, **characterised in that** cell samples are subjected where necessary to a lytic treatment in order to release the cell contents thereof.

## Revendications

1. Procédé pour la mesure in vitro de profils d'expression de gènes moyennant l'utilisation de gène spécifiques, choisis parmi le groupe consistant en les SEQ ID No 1 à SEQ ID No 1297, ainsi que de fragments de ces gènes avec au moins 20 à 2000 nucléotides, **caractérisé en ce que** l'expression de gène d'une majorité de gènes est déterminée en partant d'échantillons provenant de patients, pour déterminer la présence d'une cause infectieuse ou non infectieuse à une défaillance multiviscérale.

2. Procédé selon la revendication 1, **caractérisé en ce que** les fragments de gènes comportent 2 à 200, de préférence 20 à 80 nucléotides.

3. Procédé selon la revendication 1 ou 2, **caractérisé en ce qu'**au moins 2 à 100 gènes et/ou fragments de gènes différents sont utilisés.

4. Procédé selon l'une quelconque des revendications 1 ou 2, **caractérisé en ce qu'**au moins 200 gènes et/ou fragments de gènes différents sont utilisés.

5. Procédé selon l'une quelconque des revendications 1 ou 2, **caractérisé en ce qu'**au moins 200 à 500 gènes et/ou fragments de gènes différents sont utilisés.

6. Procédé selon l'une quelconque des revendications 1 ou 2, **caractérisé en ce qu'**au moins 500 à 1000 gènes et/ou fragments de gènes différents sont utilisés.

7. Procédé selon l'une quelconque des revendications 1 ou 2, **caractérisé en ce qu'**au moins 1000 à 2000 gènes et/ou fragments de gènes différents sont utilisés.

8. Procédé selon les revendications 1 à 7, **caractérisé en ce que** l'expression de gène est déterminée au moyen de procédés d'hybridation.

9. Procédé selon la revendication 8, **caractérisé en ce** l'expression de gène est déterminée au moyen de micromatrices.

10. Procédé selon l'une quelconque des revendications 1 à 9, **caractérisé en ce que** l'expression de gène est déterminée par des procédés indépendants de l'hybridation, en particulier par hydrolyse enzymatique et/ou chimique et/ou par des procédés d'amplification, de préférence par PCR suivie d'une quantification des acides nucléiques et/ou de dérivés et de fragments de ceux-ci.

11. Procédé selon l'une quelconque des revendications 1 à 10, **caractérisé en ce que** l'échantillon est choisi parmi : des liquides physiologiques, en particulier du sang, du liquide cérébrospinal, de l'urine, de l'épanchement par ascite, du liquide séminal, de la salive, de liquide de ponction ; des contenus de cellules ou un mélange de ceux-ci

12. Procédé selon l'une quelconque des revendications 1 à 11, **caractérisé en ce que** les échantillons de cellules sont éventuellement soumis à un traitement de lyse pour libérer leurs contenus cellulaires.
